# EUROPEAN PATENT SPECIFICATION

(11) **EP 2 124 997 B1**
(45) Date of publication and mention of the grant of the patent: **08.08.2012**
(21) Application number: 07839723.9
(22) Date of filing: 19.10.2007
(51) Int. Cl.: A61K 38/17, A61K 38/20, A61P 19/06, A61P 19/02

(54) **USE OF IL-1 ANTAGONISTS TO TREAT GOUT AND PSEUDOGOUT**
VERWENDUNG VON IL-1-ANTAGONISTEN ZUR BEHANDLUNG VON GICHT UND PSEUDO-GICHT
UTILISATION D'ANTAGONISTES IL-1 POUR TRAITER LA GOUTTE OU LA PSEUDO-GOUTTE

(30) Priority: 20.10.2006 US 853385 P
(43) Date of publication of application: 02.12.2009
(73) Proprietor: Regeneron Pharmaceuticals, Inc., Tarrytown, NY 10591 (US)
(72) Inventor: VICARY, Catherine, New York, NY 10033 (US); MELLIS, Scott, New Rochelle, NY 10804 (US)
(74) Representative: Bentham, Andrew
(86) International application number: PCT/US2007/022377
(87) International publication number: WO 2008/051496

(56) References cited:
- WO-A-2004/100987
- WO-A-2005/117945
- WO-A-2006/084145
- US-A1- 2005 129 685
- MARTINON FABIO ET AL: "Gout-associated uric acid crystals activate the NALP3 inflammasome" NATURE, NATURE PUBLISHING GROUP, LONDON, UK, vol. 440, no. 7081, 9 March 2006 (2006-03-09), pages 237-241, XP002430440 ISSN: 0028-0836
- GABAY C: "IL-1 TRAP" CURRENT OPINION IN INVESTIGATIONAL DRUGS, CURRENT DRUGS, LONDON, GB, vol. 4, no. 5, 1 May 2003 (2003-05-01), pages 593-597, XP009017868 ISSN: 0967-8298
- B. N. CRONSTEIN ET AL: "The inflammatory proces of gout and its treatment" ARHRITIS RESEARCH AND THERAPY, vol. 8, no. S1, 12 April 2006 (2006-04-12), XP002494437
- F. S. DI GIOVINE ET AL: "Interleukin 1 (IL 1) as a mediator of crystal arthritis. stimulation of T cell and Synovial Fibroblast mitogenesis by urate Crystal-induced IL 1" THE JOURNAL OF IMMUNOLOGY, vol. 138, no. 10, 15 May 1987 (1987-05-15), pages 3213-3218, XP002494438
- MATSUKAWA A ET AL: "ANALYSIS OF THE CYTOKINE NETWORK AMONG TUMOR NECROSIS FACTOR ALPHA, INTERLEUKIN-1BETA, INTERLEUKIN-8, AND INTERLEUKIN-1 RECEPTOR ANTAGONIST IN MONOSODIUM URATE CRYSTAL-INDUCED RABBIT ARTHRITIS" LABORATORY INVESTIGATION, UNITED STATES AND CANADIAN ACADEMY OF PATHOLOGY, BALTIMORE, US, vol. 78, no. 5, 1 May 1998 (1998-05-01), pages 559-569, XP009063666 ISSN: 0023-6837
- HAL M. HOFFMAN ET AL: "Prevention of cold-associated acute inflammation in familial cold autoinflammatory syndrome by interleukin-1 receptor antagonist", THE LANCET, vol. 364, 13 November 2004 (2004-11-13), pages 1779-1785,
- PHILIP N. HAWKINS ET AL: "Interleukin-1-Receptor Antagonist in teh Muckle-Wells syndrome", THE NEW ENGLAND JOURNAL OF MEDICINE, vol. 348, no. 25, 19 June 2003 (2003-06-19), pages 2583-2584,

## Description

### BACKGROUND

### Field of the Invention

The invention relates to methods of using interleukin-1 (IL-1) antagonists to treat a metabolic rheumatic disorder associated with hyperuricemia, where the disorder is gout, for instance chronic active (refractory) gout. Further, the invention also encompasses treatment of pseudogout.

### Description of Related Art

Metabolic rheumatic disorders associated with hyperuricemia, such as gout, are characterized by perversion of the purine metabolism resulting in hyperuricemia, i.e. an excess of uric acid in the blood, attacks of acute arthritis, and formation of chalky deposits in the cartilages of the joints. These deposits are made up chiefly of urates, or uric acid.

Known methods for treating gout include the use of uric acid synthesis inhibitors to inhibit the accumulation of uric acid in the body, and use of uric acid excretion promoters to accelerate the rapid excretion of uric acid accumulated in the body. Allopurinol is an example of a uric acid synthesis inhibitor. Probenecid, sulfinpyrazone and benzbromarone are examples of uric acid excretion promoters. Interleukin-6 (IL-6) has been proposed for use in the treatment of gout as a serum uric acid decreasing agent (see US Patent 6,007,804).

Pseudogout is not a hyperuremic disorder, and involves the deposition of calcium pyrophosphate.

The following documents are also acknowledged:
- WO 2006/084145, which is concerned with methods of using IL-1 antagonists to reduce C-reactive protein;
- WO 2055/117945, which is concerned with methods of suing IL-1 antagonists to treat auto-inflammatory disease;
- WO 2004/100987, which is concerned with methods of using IL-1 antagonists to treat neointimal hyperplasia;
- US 2005/0129685, which is concerned with use of IL-1 blockers to prevent corneal inflammation and neovascularization;
- Gabay et al, 2003, Current Opinion in Investigational Drugs, Current Drugs, 4(5): 593-597, which is concerned with an IL-1 trap; and
- Martion et al, 2006, Nature, Vol. 440, No. 7081: 237-241, which is concerned with how gout-associated crystals activate the NALP3 inflammasome.

### BRIEF SUMMARY OF THE INVENTION

In a first aspect, the invention features the use an interleukin 1 (IL-1) antagonist in the manufacture of a medicament for treating, inhibiting or ameliorating a metabolic rheumatic disorder associated with hyperuricemia, wherein the IL-1 antagonist is a fusion protein comprising an IL-1 binding portion of the extracellular domain of human IL-1RAcP, an IL-1 binding portion of the extraceullar domain of human IL-1R1, and a multimerizing component, and the metabolic rheumatic disorder is gout.

An IL-1 antagonist is a compound capable of blocking or inhibiting the biological action of IL-1, including fusion proteins capable of trapping IL-1, such as an IL-1 "trap". In a preferred embodiment, the IL-1 trap is an IL-1-specific fusion protein comprising two IL-1 receptor components and a multimerizing component, for example, an IL-1 trap described in U.S. patent No. 6,927,044. An IL-1 trap fusion protein comprises an IL-1 binding portion of the extracellular domain of human IL-1 RAcP, an IL-1 binding portion of the extracellular domain of human IL-1 RI, and a multimerizing component. In a specific embodiment, the IL-1 trap is the fusion protein shown in SEQ ID NO:10 (rilonacept) or a protein having at least 95% identity to the protein of SEQ ID NO:10 and capable of binding and inhibiting IL-1. Use of the IL-1 trap to treat gout offers unexpected advantages relative to the use of prior art IL-1 antagonists for several reasons, including allowing alleviation of gout symptoms with reduced frequency of administration, reduced side effects such as, for example, reduced injection site inflammation or reduced immunogenicity.

The metabolic rheumatic disorder associated with hyperuricemia is gout. The subject being treated is most preferably a human diagnosed as suffering from gout, for example, chronic active gout. The method of the invention encompasses preventing or ameliorating gout in a human subject suffering therefrom.

In a second aspect, the invention features an interleukin 1 (IL-1) antagonist for use in a method of treating, inhibiting, or ameliorating pseudogout, the method comprising administering to a subject in need a therapeutic amount of the antagonist, wherein the disorder is treated, inhibited, or ameliorated and wherein the IL-1 antagonist is a fusion protein as defined in the invention.

The invention includes administration of the IL-1 antagonist by any means known to the art, for example, subcutaneous, intramuscular, intravenous, transdermal administration or oral routes of administration. Preferably, administration is by subcutaneous or intravenous injection or intravenous infusion.

In specific embodiments, the subject is treated with a combination of an IL-1 trap and a second therapeutic agent. The second therapeutic agent an additional IL-1 antagonist and/or co-therapies such as uric acid synthesis inhibitors to inhibit the accumulation of uric acid in the body, for example, allopurinol, uric acid excretion promoters to accelerate the rapid excretion of uric acid accumulated in the body, for example, probenecid, sulfinpyrazone and/or benzbromarone are examples of uric acid excretion promoters; corticosteroids; non-steroidal anti-inflammatory drugs (NSAIDs); and/or cholchicine.

Hence, the present invention provides for the use of an IL-1 antagonist as defined in the invention as a first therapeutic agent,
and of one or more further therapeutic agents selected from another IL-1 antagonist, a corticosteroid, a non-steroidal anti-inflammatory drug (NSAID) and colchicines
In the manufacture of a medicament for treating, inhibiting, or ameliorating a metabolic rheumatic disorder associated with hyperuricemia where the metabolic rheumatic disorder is gout.

In a third aspect, the invention features the use of an interleukin 1 (IL-1) antagonist in the manufacture of a medicament for treating, inhibiting or ameliorating a metabolic rheumatic disorder associated with hyperuricemia, wherein the IL-1 antagonist is a fusion protein comprising an IL-1 binding portion of the extracellular domain of human IL-1RAcP, an IL-1 binding portion of the extraceullar domain of human IL-1R1, and a multimerizing component, and the metabolic rheumatic disorder is gout. Preferably, the IL-1 antagonist is the fusion protein show in the amino acid sequence of SEQ ID NO:10 (rilonacept). In a preferred embodiment, the metabolic rheumatic disorder is chronic active (refractory) gout. More specifically, in a preferred embodiment, rilonacept is administered subcutaneously as an initial dose of between about 250-500 mg, followed by one or more doses of 125-250 mg administered subcutaneously.

In a fourth aspect, the invention provides the use of an IL-1 antagonist as defined above as a first therapeutic agent, and of one or more further therapeutic agents selected from another IL-1 antagonist, a corticosteroid, a non-steroidal anti-inflammatory drug (NSAID) and colchicine, in the manufacture of a medicament for treating, inhibiting, or ameliorating a metabolic rheumatic disorder associated with hyperuricemia, where the metabolic rheumatic disorder is gout. Administration of the first and second therapeutic agents may be separately, simultaneously, or sequentially.

Other objects and advantages will become apparent from a review of the ensuing detailed description.

### DETAILED DESCRIPTION

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only by the appended claims.

As used in this specification and the appended claims, the singular forms "a", "an", and "the" include plural references unless the context clearly dictates otherwise. Thus for example, a reference to "a methods" includes one or more methods, and/or steps of the type described herein and/or which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

### Metabolic Rheumatic Disorders Associated with Hyperuricemia

Gout is a group of metabolic rheumatic disorders associated with hyperuricemia and is the most common cause of an inflammatory arthropathy in middle-aged men. Gout is essentially a disorder of urate metabolism. Deposition of urate crystals in hyperuricemic individuals results in acute gout, characterized by agonizing pain and inflammation of rapid onset, most frequently affecting the first metatarsophalangeal joint. Hyperuricemia is associated with an increased risk of developing gout and this increases with the degree and duration of the hyperuricemia.

Treatment of gout aims to relieve pain and inflammation of the acute attack, and reduce the incidence of recurrent attacks. Dietary and pharmacological urate-lowering therapies principally aim to prevent clinical joint damage. Common approaches to the treatment of acute gout are corticosteroids, non-steroidal anti-inflammatory drugs (NSAIDs), and colchicine. The side effects of these drugs, particularly in the frail, elderly population who experience the highest incidence of acute gout, can be serious. An approach to the prevention of recurrent gout is the use of a xanthine oxidase inhibitor, allopurinol. However, allopurinol can have serious side effects such as allopurinol hypersensitivity syndrome (see, for example, Arellano et al. (1993) Ann Pharmacother 27:337-343).

Alternative drugs for preventing gout include the uricosuric agent sulphinpyrazone, limited by its side-effect profile, and benzbromarone and probenecid. Fenofibrate, a drug well known in the treatment of various forms of hyperlipidemia, has been proposed for the treatment of hyperuricemia. **Pseudogout**

Pseudogout is a type of arthritis that, as the name implies, can cause symptoms similar to gout, but in reaction to a different type of crystal deposit. Pseudogout, sometimes referred to as calcium pyrophosphate deposition disease, can cause severe episodes of localized pain and swelling resulting in incapacitation for days or weeks. It also can cause more chronic arthritis that mimics osteoarthritis or rheumatoid arthritis. Knees are most often involved but wrists, shoulders, ankles, elbows or hands can be affected. Pseudogout is caused when deposits of calcium pyrophosphate crystals accumulate in a joint.

### IL-1 Trap Antagonists

Interleukin-1 (IL-1) traps are multimers of fusion proteins containing IL-1 receptor components and a multimerizing component capable of interacting with the multimerizing component present in another fusion protein to form a higher order structure, such as a dimer. Cytokine traps are a novel extension of the receptor-Fc fusion concept in that they include two distinct receptor components that bind a single cytokine, resulting in the generation of antagonists with dramatically increased affinity over that offered by single component reagents. In fact, the cytokine traps that are described herein are among the most potent cytokine blockers ever described. Briefly, the cytokine traps called IL-1 traps are comprised of the extracellular domain of human IL-1R Type I (IL-1 RI) or Type II (IL-1 RII) followed by the extracellular domain of human IL-1 Accessory protein (IL-1AcP), followed by a multimerizing component. In a preferred embodiment, the multimerizing component is an immunoglobulin-derived domain, such as, for example, the Fc region of human IgG. including part of the hinge region, the CH2 and CH3 domains. An immunoglobulin-derived domain may be selected from any of the major classes of immunoglobulins, including IgA, IgD, IgE, IgG and IgM, and any subclass or isotype, e.g. IgG1, IgG2, IgG3 and IgG4; IgA-1 and IgA-2. Alternatively, the IL-1 traps are comprised of the extracellular domain of human IL-1AcP, followed by the extracellular domain of human IL-1 RI or IL-1R11, followed by a multimerizing component. For a more detailed description of the IL-1 traps, see WO 00/18932. Preferred IL-1 traps have the amino acid sequence shown in SEQ ID NOs: 2, 4, 6, 8, 10, 12, 14, 16, 18, 20, 22, 24, and 26, or a substantially identical protein at least 95% identity to a sequence of SEQ ID NO:2, 4, 6, 8, 10, 12, 14, 16, 18, 20, 22, 24, or 26, and capable of binding and inhibiting IL1. Most preferably, the IL-1 antagonist is the protein of SEQ ID NO:10 (rilonacept).

In specific embodiments, the IL-1 antagonist comprises an antibody fragment capable of binding IL-1α, IL-1β, IL-1R1 and/or IL-1RAcp, or a fragment thereof. The preferred embodiment would be an antagonist of IL-1β. One embodiment of an IL-1 antagonist comprising one or more antibody fragments, for example, single chain Fv (scFv), is described in U.S. 6,472,179. In all of the IL-1 antagonist embodiments comprising one or more antibody-derived components specific for IL-1 or an IL-1 receptor, the components may be arranged in a variety of configurations, e.g., a IL-1 receptor component(s)-scFv(s)-multimerizing component; IL-1 receptor component(s)-multimerizing component-scFv(s); scFv(s)-IL-1 receptor component(s)-multimerizing component, ScFv-ScFv-Fc, etc., so long as the molecule or multimer is capable of inhibiting the biological activity of IL-1.

### Treatment Population

The instant invention provides an interleukin 1 (IL-1) antagonist for use in a method of treatment of pseudogout and metabolic rheumatic disorders associated with hyperuricemia to human patients suffering therefrom, where the metabolic rheumatic disorder is gout. The treatment population is thus human subjects diagnosed as suffering from pseudogout or gout. The invention encompasses an interleukin 1 (IL-1) antagonist for use in a method of the treatment of a human subject at risk of suffering from a recurrent gout episode or for developing gout or pseudogout.

### Methods of Administration

The invention features methods of treatment comprising administering to a subject an effective amount of an agent of the invention. In a preferred aspect, the agent is substantially purified (*e.g*., substantially free from substances that limit its effect or produce undesired side-effects).

Various delivery systems are known and can be used to administer an agent of the invention, *e.g*., encapsulation in liposomes, microparticles, microcapsules, recombinant cells capable of expressing the compound, receptor-mediated endocytosis (see, *e.g*., Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, epidural, and oral routes. The compounds may be administered by any convenient route, for example by infusion or bolus injection, by absorption through epithelial or mucocutaneous linings (*e.g.*, oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g*., by use of an inhaler or nebulizer, and formulation with an aerosolizing agent.

In a specific embodiment, it may be desirable to administer the pharmaceutical compositions of the invention locally to the area in need of treatment; this may be achieved, for example, and not by way of limitation, by local infusion during surgery, topical application, *e.g*., by injection, by means of a catheter, or by means of an implant, said implant being of a porous, non-porous, or gelatinous material, including membranes, such as sialastic membranes, fibers, commercial skin substitutes or angioplasty balloons or stents.

In another embodiment, the active agent can be delivered in a vesicle, in particular a liposome (see Langer (1990) Science 249:1527-1533). In yet another embodiment, the active agent can be delivered in a controlled release system. In one embodiment, a pump may be used (see Langer (1990) *supra*). In another embodiment, polymeric materials can be used (see Howard et al. (1989) J. Neurosurg. 71:105). In another embodiment where the active agent of the invention is a nucleic acid encoding a protein, the nucleic acid can be administered *in vivo* to promote expression of its encoded protein, by constructing it as part of an appropriate nucleic acid expression vector and administering it so that it becomes intracellular, *e.g*., by use of a retroviral vector (see, for example, U.S. Patent No. 4,980,286), or by direct injection, or by use of microparticle bombardment (*e.g.*, a gene gun; Biolistic, Dupont), or coating with lipids or cell-surface receptors or transfecting agents, or by administering it in linkage to a homeobox-like peptide which is known to enter the nucleus (see *e.g*., Joliot et al. 1991, Proc. Natl. Acad. Sci. USA 88:1864-1868), etc. Alternatively, a nucleic acid can be introduced intracellularly and incorporated within host cell DNA for expression, by homologous recombination.

### Combination Therapies

In numerous embodiments, the IL-1 antagonists of the present invention may be administered in combination with one or more additional compounds or therapies. Combination therapy may be separate, simultaneous or sequential. The IL-1 traps of the invention may be combined with, for example, TNF-inhibiting agents such as etanercept (ENBREL®, Amgen), infliximab (REMICADE®, Centocor), HUMIRA® (Abbott). Combination therapy may also include treatment with drugs currently used for the treatment or prevention of gout, including corticosteroids, non-steroidal anti-inflammatory drugs (NSAIDs); colchicine; xanthine oxidase inhibitors such as allopurinol; uricosuric agents sulphinpyrazone, benzbromarone and probenecid; and fenofibrate. Preferred co-therapeutics include NSAIDs, steroids and/or cholchicine.

### Pharmaceutical Compositions

The present invention also provides pharmaceutical compositions. Such compositions comprise a therapeutically effective amount of an active agent, and a pharmaceutically acceptable carrier. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin.

In a preferred embodiment, the composition is formulated in accordance with routine procedures as a pharmaceutical composition adapted for intravenous administration to human beings. Where necessary, the composition may also include a solubilizing agent and a local anesthetic such as lidocaine to ease pain at the site of the injection. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The active agents of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the active agent of the invention which will be effective in the treatment of delayed-type hypersensitivity can be determined by standard clinical techniques based on the present description. In addition, *in vitro* assays may optionally be employed to help identify optimal dosage ranges. The precise dose to be employed in the formulation will also depend on the route of administration, and the seriousness of the condition, and should be decided according to the judgment of the practitioner and each subject's circumstances.

For systemic administration, a therapeutically effective dose can be estimated initially from *in vitro* assays. For example, a dose can be formulated in animal models to achieve a circulating concentration range that includes the IC₅₀ as determined in cell culture. Such information can be used to more accurately determine useful doses in humans. Initial dosages can also be estimated from *in vivo* data, e.g., animal models, using techniques that are well known in the art. One having ordinary skill in the art could readily optimize administration to humans based on animal data.

### EXAMPLES

The following example is put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. A Phase I/II, Placebo Controlled Pilot Study of the Safety, Tolerability, and Clinical Activity of Rilonacept for the Treatment of Chronic Active Gout

Gout is a common disease with increasing incidence. There are approximately 5MM Americans with gout. Medical needs are not fully met; a large number of individuals are either intolerant or not good candidates for current therapeutic or prophylactic strategies. This study explores the activity of rilonacept at one end of the gout spectrum: chronic active (refractory) gout. Results from this study may or may not be indicative of rilonacept's potential utility in acute active gout or in the prevention of gout flares; however, activity in this situation suggests potential benefit. The chronic active (refractory) gout population, while small, represents a true situation of medical need.

Primary Objective: To assess the safety of rilonacept in subjects with chronic, active gout, having at least one joint continuously inflamed.

Secondary Obiectives: (1) To compare within subject changes in self-reported pain score (VAS) during the placebo run-in phase with the active treatment phase; (2) to assess the changes in the Subject and Physician Global Assessments during the placebo run-in and active treatment phases; (3) to assess the effect of rilonacept on C-reactive protein (CRP) and erythrocyte sedimentation rate (ESR) in subjects with chronic active gout.

Study Arms and Cohorts: Subjects are screened at Day -7; a two-week single blind placebo run-in begins at the Baseline visit (2 x 2 ml of placebo for rilonacept SQ); Single blind rilonacept 320 mg is administered subcutaneously at the Week 2 visit and then subjects self-inject 160 mg/week at home. Visits occur every two weeks through Week 8. PPD skin test, CXR, and inclusion/exclusion criteria are assessed at the Screening visit. Self-injection technique is taught at Screening and Baseline. Laboratory sample collections occur at Screening, Baseline, Weeks 2, 4, and 8. A follow-up visit occurs at Week 14.

Sample Size and Number of Sites: N = 5 from up to 5 sites in the U.S.

Drug Supply: Placebo. 5 subjects x 2 vials = 10 vials + 30% overage = 13 vials; Rilonacept: 5 subjects x 8 vials = 48 vials + 30% overage = 66 vials, 3 vials per kit.

Subiect Eligibility Criteria. Inclusion Criteria: (1) Male or female ≥ 21 years; (2) Chronis, active monoarticular or polyarticular gout (≥ 1 continuously inflamed joint due to gout, ± tophi); (3) VAS 10-point pain scale score of ≥ 3 (i.e., moderate or greater) due to joint pain/inflammation; (4) subjects for whom standard therapies are ineffective or associated with risks related to side effects.

Exclusion Criteria: (1) Organ transplant recipient; (2) current active infection; (3) serum creatinine < 2.5 mg/dL; (4) other arthritic condition that could interfere with evaluations.

Primary Endpoint: Tolerability and safety profile of rilonacept.

Secondary Endpoints: (1) The change from Baseline to Week 8 in subject's pain score (10 cm VAS scale); (2) the change from Baseline to Week 8 in the Subject's Global Assessment; (3) the change from Baseline to Week 8 in the Physician's Global Assessment; (4) the change from Baseline to Week 8 in ESR and CRP.

### Example 2. Safety of Rilonacept in Subjects with Chronic Active Gouty Arthritis.

A 14 week, multi-center, non-randomized, single-blind, placebo-controlled, monosequence crossover study of rilonacept in subjects with chronic, active monoarticular or polyarticular gouty arthritis. Subjects receive 2 weeks of single-blind placebo followed by a loading dose of subcutaneous injections of 320 mg rilonacept, followed by weekly subcutaneous injections for 5 weeks of 160 mg rilonacept.Study Population. The study population included adult subjects (at least 18 years of age, male or female) with chronic, active monoarticular or polyarticular gouty arthritis diagnosed by a physician for at least 6 months with at least one continuously inflamed joint (self-reported or otherwise) for ≥ 4 weeks, a diagnosis of gout based on a history of the presence of crystals in the synovial fluid analysis, chronically elevated serum uric acid, and/or tophi; a visual analogue scale increment pain scale score of at least 3 due to joint pain/inflammation at both the Screening and Baseline Visits, and subjects for whom standard therapies for gout are less than effective or are associated with risks related to side effects.

Study Design. This study was a 14-week, multi-center, non-randomized, single-blind, placebo-controlled, monosequence crossover study of IL-1 trap (rilonacept) in subjects with chronic, active monoarticular or polyarticular gouty arthritis. Subjects received 2 weeks of single-blind placebo followed by a loading dose of subcutaneous injections of 320 mg rilonacept, followed by weekly subcutaneous injections for 5 weeks of 160 mg rilonacept. The study was conducted in approximately 12 sites in the U.S. This study includes a monosequence crossover design for the enrolled subjects: Treatment 1: Placebo injections for two weeks; Treatment 2: Injections of rilonacept for six weeks. Descriptive statistics was used to evaluate safety and efficacy of rilonacept in gout. Approximately 10 subjects were enrolled to receive placebo (2 weeks) and rilonacept (6 weeks) administered subcutaneously. Subjects received a total of two doses of placebo (on study days 0 and 7) and six doses of rilonacept on Days 14, 21, 28, 35, 42, and 49 during the study. Dose escalation was not allowed. Subjects were evaluated for efficacy and safety on a regular basis with clinical observations and laboratory measurements including anti- rilonacept antibodies, hs-CRP and ESR. The overall structure of the study included the following periods: Screening period: Screening procedures occurred within 7 days of start of study and included obtaining informed consent and evaluations to determine eligibility for study participation. Baseline: At the baseline visit (day 0), eligibility was confirmed, and the subject enrolled. Baseline assessments were made. The first injection of placebo was administered, and a vial of placebo dispensed. Placebo Treatment period: During the treatment period (Day 0 through Week 2), patients received placebo study medication, efficacy assessments were taken; safety and tolerability assessments were taken, including urine and blood samples for clinical laboratory testing. Active Treatment period: During the treatment period (Week 2 through Week 8), patients received active study medication, efficacy assessments were taken; safety and tolerability assessments were taken, including urine and blood samples for clinical laboratory testing. Blood samples were collected for biomarkers, IL-1 trap (rilonacept) plasma levels, and rilonacept antibodies. Follow-up: At Week 14, vital signs, bodyweight, adverse events, and concomitant medications assessments were taken. Blood samples were collected for biomarkers, rilonacept plasma levels, and rilonacept antibodies. The results are shown in Table 1. The first column lists the parameters assessed; column 2 (placebo response) compares the parameter measurements obtained at Week 2 with those measured at Day 0; column 3 (response of rilonacept) compares the parameter measurements obtained at Week 8 with those of Week 2; and column 4 (effect of withdrawal from treatment with rilonacept) compares the parameter measurements obtained at Week 14 with those obtained at Week 2.

Results. -During treatment with placebo, there was no apparent trend toward improvement in any clinical parameter nor in CRP. Also, during treatment with placebo, there is no evidence of improvement. At the end of treatment with rilonacept, the proportion of responders is significantly better than a placebo response of 10%, regardless of how response was defined (p<0.01). Also, there was a significant reduction in patient's self-reported pain (p = 0.02). When treatment with rilonacept was withdrawn, the trends toward efficacy waned and pain returned. These results suggest that placebo response is minimal, and reduction in pain is not observed until treatment with rilonacept is administered.

**Table 1**

| **Assessment** | **Placebo Response** | **Rilonacept Response** | | | **Effects of Withdrawal from Treatment with Rilonacept** |
|---|---|---|---|---|---|
| | **Change Day 0- week 2** | **Change Week 2-4** | **Change Week 2-4** | **Change Week 2-8** | **Change Week 8-14** |
| **Subject's Assessment of Pain** | 0.96 | 0.046 | 0.07 | 0.02 | 0.07 |
| **Physician's Global Assessment** | 0.09 | 0.4 | 0.8 | 0.2 | 0.02 |
| **Subject's Global Assessment** | 1.0 | 0.07 | 0.14 | 0.06 | 0.02 |
| **Number of Affected Joints** | 0.3 | 0.7 | 0.9 | 0.099 | 0.95 |
| **Symptom-Adjusted Scores for Affected Joints (maximum of 3 per joint)** | 0.2 | 0.7 | 0.8 | 0.04 | 0.3 |
| **Severity-and-Symptom-Adjusted Scores for Affected Joints (maximum of 9 per joint)** | 0.2 | 0.2 | 0.8 | 0.04 | 0.14 |
| **Change from Baseline CRP** | 0.5 | 0.002 | 0.004 | 0.004 | 0.04 |

## Claims

1. Use of an interleukin 1 (IL-1) antagonist in the manufacture of a medicament for treating, inhibiting or ameliorating a metabolic rheumatic disorder associated with hyperuricemia, wherein the IL-1 antagonist is a fusion protein comprising an IL-1 binding portion of the extracellular domain of human IL-1RAcP, an IL-1 binding portion of the extraceullar domain of human IL-1R1, and a multimerizing component, and the metabolic rheumatic disorder is gout.

2. Use according to claim 1, wherein the metabolic rheumatic disorder is chronic active (refractory) gout.

3. Use according to claim 1, wherein the IL-1 antagonist is a fusion protein comprising the amino acid sequence of SEQ ID NO:10.

4. Use according to any one of claim 1 to 3, wherein administration is by subcutaneous or intravenous administration.

5. Use according to claim 4, wherein administration is by single or multiple subcutaneous injections or intravenous infusions.

6. Use according to claim 5, wherein administration comprises an initial dose of between about 250-500 mg of the IL-1 antagonist administered subcutaneously, followed by one or more doses of 125-250 mg administered subcutaneously.

7. Use of an IL-1 antagonist as defined in claim 1 or 3 as a first therapeutic agent,
and of one or more further therapeutic agents selected from another IL-1 antagonist, a corticosteroid, a non-steroidal anti-inflammatory drug (NSAID) and colchicine
in the manufacture of a medicament for treating, inhibiting, or ameliorating a metabolic rheumatic disorder associated with hyperuricemia where the metabolic rheumatic disorder is gout.

8. An interleukin 1 (IL-1) antagonist for use in a method of treating, inhibiting, or ameliorating pseudogout, the method comprising administering to a subject in need a therapeutic amount of the antagonist, wherein the disorder is treated, inhibited, or ameliorated and wherein the IL-1 antagonist is a fusion protein as defined in claim 1 or 3.

9. An interleukin 1 (IL-1) antagonist for use in a method of treating, inhibiting or ameliorating a metabolic rheumatic disorder associated with hyperuricemia, wherein the IL-1 antagonist is a fusion protein as defined in claim 1 or 3 and the metabolic rheumatic disorder is gout.

10. Use of an IL-1 antagonist as defined in claim 1 or 3 in the manufacture of a medicament for the treatment, in combination with one or more further therapeutic agents selected from another IL-1 antagonist, a corticosteroid, a non-steroidal anti-inflammatory drug (NSAID) and colchicine, of a metabolic rheumatic disorder associated with hyperuricemia, where the metabolic rheumatic disorder is gout.

11. Use of one of more therapeutic agents selected from an IL-1 antagonist other than that defined in claim 1, a corticosteroid, a non-steroidal anti-inflammatory drug (NSAID) and colchicine in the manufacture of a medicament for the treatment, in combination with an IL-1 antagonist as defined in claim 1 or 3, of a metabolic rheumatic disorder associated with hyperuricemia, where the metabolic rheumatic disorder is gout.

12. A product containing an IL-1 antagonist as defined in claim 1 or 3 and one or more further therapeutic agents selected from another IL-1 antagonist, a corticosteroid, a non-steroidal anti-inflammatory drug (NSAID) and colchicine for simultaneous, separate or sequential use in the treatment of a metabolic rheumatic disorder associated with hyperuricemia, where the metabolic rheumatic disorder is gout.

## Patentansprüche

1. Verwendung eines Interleukin-1 (IL-1)-Antagonisten bei der Herstellung eines Medikaments zur Behandlung, Inhibierung oder Besserung einer metabolischen rheumatischen Störung, die mit Hyperurikämie assoziiert ist, wobei der IL-1-Antagonist ein Fusionsprotein ist, das einen IL-1-bindenden Abschnitt der extrazellulären Domäne von humanem IL-1RAcP, einen IL-1-bindenden Abschnitt der extrazellulären Domäne von humanem IL-1R1 und eine multimerisierende Komponenten umfasst, und die metabolische rheumatische Störung Gicht ist.

2. Verwendung gemäß Anspruch 1, wobei die metabolische rheumatische Störung chronische aktive (refraktäre) Gicht ist.

3. Verwendung gemäß Anspruch 1, wobei der IL-1-Antagonist ein Fusionsprotein ist, das die Aminosäuresequenz von SEQ ID NO:10 umfasst.

4. Verwendung gemäß einem der Ansprüche 1 bis 3, wobei eine Verabreichung durch subkutane oder intravenöse Verabreichung erfolgt.

5. Verwendung gemäß Anspruch 4, wobei die Verabreichung durch eine einzelne oder durch mehrfache subkutane Injektionen oder intravenöse Infusionen erfolgt.

6. Verwendung gemäß Anspruch 5, wobei eine Verabreichung eine Anfangsdosis von zwischen etwa 250-500 mg des IL-1-Antagonisten, der subkutan verabreicht wird, gefolgt von einer oder mehreren Dosen von 125-250 mg, subkutan verabreicht, umfasst.

7. Verwendung eines IL-1-Antagonisten, wie in Anspruch 1 oder 3 definiert, als erstes therapeutisches Mittel und eines oder mehrerer weiterer therapeutischer Mittel, ausgewählt aus einem anderen IL-1-Antagonisten, einem Corticosteroid, einem nichtsteroidalen Antirheumatikum (NSAR) und Colchicin
bei der Herstellung eines Medikaments zur Behandlung, Inhibierung oder Besserung einer metabolischen rheumatischen Störung, die mit Hyperurikämie assoziiert ist, wobei die metabolische rheumatische Störung Gicht ist.

8. Interleukin-1 (IL-1)-Antagonist zur Verwendung in einem Verfahren zur Behandlung, Inhibierung oder Besserung von Pseudogicht, wobei das Verfahren Verabreichen einer therapeutischen Menge des Antagonisten an ein Subjekt, das Bedarf dafür hat, umfasst, wobei die Störung behandelt, inhibiert oder verbessert wird und wobei der IL-1-Antagonist ein Fusionsprotein, wie in Anspruch 1 oder 3 definiert, ist.

9. Interleukin-1 (IL-1)-Antagonist zur Verwendung in einem Verfahren zur Behandlung, Inhibierung oder Besserung einer metabolischen rheumatischen Störung, die mit Hyperurikämie assoziiert ist, wobei der IL-1-Antagonist ein Fusionsprotein, wie in Anspruch 1 oder 3 definiert, ist und die metabolische rheumatische Störung Gicht ist.

10. Verwendung eines IL-1-Antagonisten, wie in Anspruch 1 oder 3 definiert, bei der Herstellung eines Medikaments für die Behandlung, in Kombination mit einem oder mehreren weiteren therapeutischen Mitteln, ausgewählt aus einem anderen IL-1-Antagonisten, einem Corticosteroid, einem nichtsteroidalen Antirheumatikum (NSAR) und Colchicin, einer metabolischen rheumatischen Störung, die mit Hyperurikämie assoziiert ist, wobei die metabolische rheumatische Störung Gicht ist.

11. Verwendung eines oder mehrere therapeutischer Mittel, ausgewählt aus einem anderen IL-1-Antagonisten als dem, der in Anspruch 1 definiert ist, einem Corticosteroid, einem nichtsteroidalen Antirheumatikum (NSAR) und Colchicin, bei der Herstellung eines Medikaments für die Behandlung, in Kombination mit einem IL-1-Antagonisten, wie in Anspruch 1 oder 3 definiert, einer metabolischen rheumatischen Störung, die mit Hyperurikämie assoziiert ist, wobei die metabolische rheumatische Störung Gicht ist.

12. Produkt, das einen IL-1-Antagonisten, wie in Anspruch 1 oder 3 definiert, und ein oder mehrere weitere therapeutische Mittel, ausgewählt aus einem anderen IL-1-Antagonisten, einem Corticosteroid, einem nichtsteroidalen Antirheumatikum (NSAR) und Colchicin, enthält, zur gleichzeitigen, getrennten oder sequentiellen Verwendung bei der Behandlung einer metabolischen rheumatischen Störung, die mit Hyperurikämie assoziiert ist, wobei die metabolische rheumatische Störung Gicht ist.

## Revendications

1. Utilisation d'un antagoniste de l'IL-1 (interleukine 1) dans la fabrication d'un médicament conçu pour traiter, inhiber ou améliorer une affection rhumatismale métabolique associée à une hyperuricémie, pour laquelle l'antagoniste d'IL-1 est une protéine de fusion comprenant une partie se liant à l'IL-1 du domaine extracellulaire de la protéine IL-1RAcP humaine, une partie se liant à l'IL-1 du domaine extracellulaire du récepteur IL-1R1 humain, et une composante de multimérisation, et l'affection rhumatismale métabolique est la goutte.

2. Utilisation conforme à la revendication 1, pour laquelle l'affection rhumatismale métabolique est la goutte chronique active (réfractaire).

3. Utilisation conforme à la revendication 1, pour laquelle l'antagoniste d'IL-1 est une protéine de fusion comportant la séquence d'acides aminés présentée en tant que Séquence N° 10.

4. Utilisation conforme à l'une des revendications 1 à 3, pour laquelle l'administration doit être effectuée par voie sous-cutanée ou intraveineuse.

5. Utilisation conforme à la revendication 4, pour laquelle l'administration doit être effectuée en une seule ou plusieurs injection(s) sous-cutanée(s) ou par perfusion intraveineuse.

6. Utilisation conforme à la revendication 5, pour laquelle l'administration comporte une dose initiale d'à peu près 250 à 500 mg de l'antagoniste d'IL-1, administrée par voie sous-cutanée, suivie d'une ou de plusieurs dose(s) d'à peu près 125 à 250 mg, administrée(s) par voie sous-cutanée.

7. Utilisation
- d'un antagoniste d'IL-1, tel que défini dans la revendication 1 ou 3, en tant que premier agent thérapeutique,
- et d'un ou plusieurs autre(s) agent(s) thérapeutique(s) choisi(s) parmi un autre antagoniste d'IL-1, un corticostéroïde, un médicament AINS (anti-inflammatoire non-stéroïdien), et la colchicine,
dans la fabrication d'un médicament conçu pour traiter, inhiber ou améliorer une affection rhumatismale métabolique associée à une hyperuricémie, pour laquelle l'affection rhumatismale métabolique est la goutte.

8. Antagoniste de l'IL-1 (interleukine 1) pour utilisation dans un procédé permettant de traiter, d'inhiber ou d'améliorer la pseudogoutte, lequel procédé comprend le fait d'administrer à un sujet qui en a besoin ledit antagoniste en une quantité thérapeutique, grâce à quoi le trouble est traité, inhibé ou amélioré, et lequel antagoniste d'IL-1 est une protéine de fusion telle que définie dans la revendication 1 ou 3.

9. Antagoniste de l'IL-1 (interleukine 1) pour utilisation dans un procédé permettant de traiter, d'inhiber ou d'améliorer une affection rhumatismale métabolique associée à une hyperuricémie, lequel antago-niste d'IL-1 est une protéine de fusion telle que définie dans la revendication 1 ou 3, et laquelle affection rhumatismale métabolique est la goutte.

10. Utilisation d'un antagoniste d'IL-1, tel que défini dans la revendication 1 ou 3, dans la fabrication d'un médicament conçu pour le traitement, en association avec un ou plusieurs autre(s) agent(s) thérapeutique(s) choisi(s) parmi un autre antagoniste d'IL-1, un corticostéroïde, un médicament AINS (anti-inflammatoire non-stéroïdien) et la colchicine, d'une affection rhumatismale métabolique associée à une hyperuricémie, pour laquelle l'affection rhumatismale métabolique est la goutte.

11. Utilisation d'un ou de plusieurs agent(s) thérapeutique(s) choisi(s) parmi un antagoniste d'IL-1 autre que celui défini dans la revendication 1, un corticostéroïde, un médicament AINS (anti-inflammatoire non-stéroïdien) et la colchicine, dans la fabrication d'un médicament conçu pour le traitement, en association avec un antagoniste d'IL-1 tel que défini dans la revendication 1 ou 3, d'une affection rhumatismale métabolique associée à une hyperuricémie, pour laquelle l'affection rhumatismale métabolique est la goutte.

12. Produit contenant un antagoniste d'IL-1, tel que défini dans la revendication 1 ou 3, et un ou plusieurs autre(s) agent(s) thérapeutique(s) choisi(s) parmi un autre antagoniste d'IL-1, un corticostéroïde, un médicament AINS (anti-inflammatoire non-stéroïdien) et la colchicine, pour utilisation simultanée, séparée ou successive dans le traitement d'une affection rhumatismale métabolique associée à une hyperuricémie, laquelle affection rhumatismale métabolique est la goutte.


### SEQUENCE LISTING

<110> Regeneron Pharmaceuticals, Inc.
<120> Use of IL-1 Antagonists to Treat Gout and Pseudogout
<130> 5060A-WO
<140> to be assigned
   <141> 2006-10-19
<150> 60/853, 385
   <151> 2006-10-20
<160> 26
<170> FastSEQ for windows Version 3.0
<210> 1
   <211> 2733
   <212> DNA
   <213> Homo sapiens
<400> 1
   atg gtg ctt ctg tgg tgt gta gtg agt ctc tac ttt tat gga atc ctg      48
   caa agt gat gcc tca gaa cgc tgc gat gac tgg gga cta gac acc atg      96
   agg caa atc caa gtg ttt gaa gat gag cca gct cgc atc aag tgc cca     144
   ctc ttt gaa cac ttc ttg aaa ttc aac tac agc aca gcc cat tca gct     192
   ggc ctt act ctg atc tgg tat tgg act agg cag gac cgg gac ctt gag     240
   gag cca att aac ttc cgc ctc ccc gag aac cgc att agt aag gag aaa     288
   gat gtg ctg tgg ttc cgg ccc act ctc ctc aat gac act ggc aac tat     336
   acc tgc atg tta agg aac act aca tat tgc agc aaa gtt gca ttt ccc     384
   ttg gaa gtt gtt caa aaa gac agc tgt ttc aat tcc ccc atg aaa ctc     432
   cca gtg cat aaa ctg tat ata gaa tat ggc att cag agg atc act tgt     480
   cca aat gta gat gga tat ttt cct tcc agt gtc aaa ccg act atc act     528
   tgg tat atg ggc tgt tat aaa ata cag aat ttt aat aat gta ata ccc     576
   gaa ggt atg aac ttg agt ttc ctc att gcc tta att tca aat aat gga     624
   aat tac aca tgt gtt gtt aca tat cca gaa aat gga cgt acg ttt cat     672
   ctc acc agg act ctg act gta aag gta gta ggc tct cca aaa aat gca     720
   gtg ccc cct gtg atc cat tca cct aat gat cat gtg gtc tat gag aaa     768
   gaa cca gga gag gag cta ctc att ccc tgt acg gtc tat ttt agt ttt     816
   ctg atg gat tct cgc aat gag gtt tgg tgg acc att gat gga aaa aaa     864
   cct gat gac atc act att gat gtc acc att aac gaa agt ata agt cat     912
   agt aga aca gaa gat gaa aca aga act cag att ttg agc atc aag aaa     960
   gtt acc tct gag gat ctc aag cgc agc tat gtc tgt cat gct aga agt    1008
   gcc aaa ggc gaa gtt gcc aaa gca gcc aag gtg aag cag aaa gtg cca    1056
   gct cca aga tac aca gtg tcc ggt ggc gcg cct atg ctg agc gag gct    1104
   gat aaa tgc aag gaa cgt gaa gaa aaa ata att tta gtg tca tct gca    1152
   aat gaa att gat gtt cgt ccc tgt cct ctt aac cca aat gaa cac aaa    1200
   ggc act ata act tgg tat aag gat gac agc aag aca cct gta tct aca    1248
   gaa caa gcc tcc agg att cat caa cac aaa gag aaa ctt tgg ttt gtt    1296
   cct gct aag gtg gag gat tca gga cat tac tat tgc gtg gta aga aat    1344
   tca tct tac tgc ctc aga att aaa ata agt gca aaa ttt gtg gag aat    1392
   gag cct aac tta tgt tat aat gca caa gcc ata ttt aag cag aaa cta    1440
   ccc gtt gca gga gac gga gga ctt gtg tgc cct tat atg gag ttt ttt    1488
   aaa aat gaa aat aat gag tta cct aaa tta cag tgg tat aag gat tgc    1536
   aaa cct cta ctt ctt gac aat ata cac ttt agt gga gtc aaa gat agg    1584
   ctc atc gtg atg aat gtg gct gaa aag cat aga ggg aac tat act tgt    1632
   cat gca tcc tac aca tac ttg ggc aag caa tat cct att acc cgg gta    1680
   ata gaa ttt att act cta gag gaa aac aaa ccc aca agg cct gtg att    1728
   gtg agc cca gct aat gag aca atg gaa gta gac ttg gga tcc cag ata    1776
   caa ttg atc tgt aat gtc acc ggc cag ttg agt gac att gct tac tgg    1824
   aag tgg aat ggg tca gta att gat gaa gat gac cca gtg cta ggg gaa    1872
   gac tat tac agt gtg gaa aat cct gca aac aaa aga agg agt acc ctc    1920
   atc aca gtg ctt aat ata tcg gaa att gag agt aga ttt tat aaa cat    1968
   cca ttt acc tgt ttt gcc aag aat aca cat ggt ata gat gca gca tat    2016
   atc cag tta ata tat cca gtc act aat tcc gga gac aaa act cac aca    2064
   tgc cca ccg tgc cca gca cct gaa ctc ctg ggg gga ccg tca gtc ttc    2112
   ctc ttc ccc cca aaa ccc aag gac acc ctc atg atc tcc cgg acc cct    2160
   gag gtc aca tgc gtg gtg gtg gac gtg agc cac gaa gac cct gag gtc    2208
   aag ttc aac tgg tac gtg gac ggc gtg gag gtg cat aat gcc aag aca    2256
   aag ccg cgg gag gag cag tac aac agc acg tac cgt gtg gtc agc gtc    2304
   ctc acc gtc ctg cac cag gac tgg ctg aat ggc aag gag tac aag tgc    2352
   aag gtc tcc aac aaa gcc ctc cca gcc ccc atc gag aaa acc atc tcc    2400
   aaa gcc aaa ggg cag ccc cga gaa cca cag gtg tac acc ctg ccc cca    2448
   tcc cgg gag gag atg acc aag aac cag gtc agc ctg acc tgc ctg gtc    2496
   aaa ggc ttc tat ccc agc gac atc gcc gtg gag tgg gag agc aat ggg    2544
   cag ccg gag aac aac tac aag acc acg cct ccc gtg ctg gac tcc gac    2592
   ggc tcc ttc ttc ctc tat agc aag ctc acc gtg gac aag agc agg tgg    2640
   cag cag ggg aac gtc ttc tca tgc tcc gtg atg cat gag gct ctg cac    2688
   aac cac tac acg cag aag agc ctc tcc ctg tct ccg ggt aaa            2730
   tga                                                                2733
<210> 2
   <211> 910
   <212> PRT
   <213> Homo sapiens
<400> 2
<210> 3
   <211> 2703
   <212> DNA
   <213> Homo sapiens
<400> 3
   atggtgttac tcagacttat ttgtttcata gctctactga tttcttctct ggaggctgat      60
   aaatgcaagg aacgtgaaga aaaaataatt ttagtgtcat ctgcaaatga aattgatgtt     120
   cgtccctgtc ctcttaaccc aaatgaacac aaaggcacta taacttggta taaggatgac     180
   agcaagacac ctgtatctac agaacaagcc tccaggattc atcaacacaa agagaaactt     240
   tggtttgttc ctgctaaggt ggaggattca ggacattact attgcgtggt aagaaattca     300
   tcttactgcc tcagaattaa aataagtgca aaatttgtgg agaatgagcc taacttatgt     360
   tataatgcac aagccatatt taagcagaaa ctacccgttg caggagacgg aggacttgtg     420
   tgcccttata tggagttttt taaaaatgaa aataatgagt tacctaaatt acagtggtat     480
   aaggattgca aacctctact tcttgacaat atacacttta gtggagtcaa agataggctc     540
   atcgtgatga atgtggctga aaagcataga gggaactata cttgtcatgc atcctacaca     600
   tacttgggca agcaatatcc tattacccgg gtaatagaat ttattactct agaggaaaac     660
   aaacccacaa ggcctgtgat tgtgagccca gctaatgaga caatggaagt agacttggga     720
   tcccagatac aattgatctg taatgtcacc ggccagttga gtgacattgc ttactggaag     780
   tggaatgggt cagtaattga tgaagatgac ccagtgctag gggaagacta ttacagtgtg     840
   gaaaatcctg caaacaaaag aaggagtacc ctcatcacag tgcttaatat atcggaaatt     900
   gagagtagat tttataaaca tccatttacc tgttttgcca agaatacaca tggtatagat     960
   gcagcatata tccagttaat atatccagtc actaattcag aacgctgcga tgactgggga    1020
   ctagacacca tgaggcaaat ccaagtgttt gaagatgagc cagctcgcat caagtgccca    1080
   ctctttgaac acttcttgaa attcaactac agcacagccc attcagctgg ccttactctg    1140
   atctggtatt ggactaggca ggaccgggac cttgaggagc caattaactt ccgcctcccc    1200
   gagaaccgca ttagtaagga gaaagatgtg ctgtggttcc ggcccactct cctcaatgac    1260
   actggcaact atacctgcat gttaaggaac actacatatt gcagcaaagt tgcatttccc    1320
   ttggaagttg ttcaaaaaga cagctgtttc aattccccca tgaaactccc agtgcataaa    1380
   ctgtatatag aatatggcat tcagaggatc acttgtccaa atgtagatgg atattttcct    1440
   tccagtgtca aaccgactat cacttggtat atgggctgtt ataaaataca gaattttaat    1500
   aatgtaatac ccgaaggtat gaacttgagt ttcctcattg ccttaatttc aaataatgga    1560
   aattacacat gtgttgttac atatccagaa aatggacgta cgtttcatct caccaggact    1620
   ctgactgtaa aggtagtagg ctctccaaaa aatgcagtgc cccctgtgat ccattcacct    1680
   aatgatcatg tggtctatga gaaagaacca ggagaggagc tactcattcc ctgtacggtc    1740
   tattttagtt ttctgatgga ttctcgcaat gaggtttggt ggaccattga tggaaaaaaa    1800
   cctgatgaca tcactattga tgtcaccatt aacgaaagta taagtcatag tagaacagaa    1860
   gatgaaacaa gaactcagat tttgagcatc aagaaagtta cctctgagga tctcaagcgc    1920
   agctatgtct gtcatgctag aagtgccaaa ggcgaagttg ccaaagcagc caaggtgaag    1980
   cagaaagtgc cagctccaag atacacagtg gaatccggag acaaaactca cacatgccca    2040
   ccgtgcccag cacctgaact cctgggggga ccgtcagtct tcctcttccc cccaaaaccc    2100
   aaggacaccc tcatgatctc ccggacccct gaggtcacat gcgtggtggt ggacgtgagc    2160
   cacgaagacc ctgaggtcaa gttcaactgg tacgtggacg gcgtggaggt gcataatgcc    2220
   aagacaaagc cgcgggagga gcagtacaac agcacgtacc gtgtggtcag cgtcctcacc    2280
   gtcctgcacc aggactggct gaatggcaag gagtacaagt gcaaggtctc caacaaagcc    2340
   ctcccagccc ccatcgagaa aaccatctcc aaagccaaag ggcagccccg agaaccacag    2400
   gtgtacaccc tgcccccatc ccgggaggag atgaccaaga accaggtcag cctgacctgc    2460
   ctggtcaaag gcttctatcc cagcgacatc gccgtggagt gggagagcaa tgggcagccg    2520
   gagaacaact acaagaccac gcctcccgtg ctggactccg acggctcctt cttcctctat    2580
   agcaagctca ccgtggacaa gagcaggtgg cagcagggga acgtcttctc atgctccgtg    2640
   atgcatgagg ctctgcacaa ccactacacg cagaagagcc tctccctgtc tccgggtaaa    2700
   tga                                                                  2703
<210> 4
   <211> 900
   <212> PRT
   <213> Homo sapiens
<400> 4
<210> 5
   <211> 2709
   <212> DNA
   <213> Homo sapiens
<400> 5
   atggtgttac tcagacttat ttgtttcata gctctactga tttcttctct ggaggctgat      60
   aaatgcaagg aacgtgaaga aaaaataatt ttagtgtcat ctgcaaatga aattgatgtt     120
   cgtccctgtc ctcttaaccc aaatgaacac aaaggcacta taacttggta taaggatgac     180
   agcaagacac ctgtatctac agaacaagcc tccaggattc atcaacacaa agagaaactt     240
   tggtttgttc ctgctaaggt ggaggattca ggacattact attgcgtggt aagaaattca     300
   tcttactgcc tcagaattaa aataagtgca aaatttgtgg agaatgagcc taacttatgt     360
   tataatgcac aagccatatt taagcagaaa ctacccgttg caggagacgg aggacttgtg     420
   tgcccttata tggagttttt taaaaatgaa aataatgagt tacctaaatt acagtggtat     480
   aaggattgca aacctctact tcttgacaat atacacttta gtggagtcaa agataggctc     540
   atcgtgatga atgtggctga aaagcataga gggaactata cttgtcatgc atcctacaca     600
   tacttgggca agcaatatcc tattacccgg gtaatagaat ttattactct agaggaaaac     660
   aaacccacaa ggcctgtgat tgtgagccca gctaatgaga caatggaagt agacttggga     720
   tcccagatac aattgatctg taatgtcacc ggccagttga gtgacattgc ttactggaag     780
   tggaatgggt cagtaattga tgaagatgac ccagtgctag gggaagacta ttacagtgtg     840
   gaaaatcctg caaacaaaag aaggagtacc ctcatcacag tgcttaatat atcggaaatt     900
   gagagtagat tttataaaca tccatttacc tgttttgcca agaatacaca tggtatagat     960
   gcagcatata tccagttaat atatccagtc actaattcag aacgctgcga tgactgggga    1020
   ctagacacca tgaggcaaat ccaagtgttt gaagatgagc cagctcgcat caagtgccca    1080
   ctctttgaac acttcttgaa attcaactac agcacagccc attcagctgg ccttactctg    1140
   atctggtatt ggactaggca ggaccgggac cttgaggagc caattaactt ccgcctcccc    1200
   gagaaccgca ttagtaagga gaaagatgtg ctgtggttcc ggcccactct cctcaatgac    1260
   actggcaact atacctgcat gttaaggaac actacatatt gcagcaaagt tgcatttccc    1320
   ttggaagttg ttcaaaaaga cagctgtttc aattccccca tgaaactccc agtgcataaa    1380
   ctgtatatag aatatggcat tcagaggatc acttgtccaa atgtagatgg atattttcct    1440
   tccagtgtca aaccgactat cacttggtat atgggctgtt ataaaataca gaattttaat    1500
   aatgtaatac ccgaaggtat gaacttgagt ttcctcattg ccttaatttc aaataatgga    1560
   aattacacat gtgttgttac atatccagaa aatggacgta cgtttcatct caccaggact    1620
   ctgactgtaa aggtagtagg ctctccaaaa aatgcagtgc cccctgtgat ccattcacct    1680
   aatgatcatg tggtctatga gaaagaacca ggagaggagc tactcattcc ctgtacggtc    1740
   tattttagtt ttctgatgga ttctcgcaat gaggtttggt ggaccattga tggaaaaaaa    1800
   cctgatgaca tcactattga tgtcaccatt aacgaaagta taagtcatag tagaacagaa    1860
   gatgaaacaa gaactcagat tttgagcatc aagaaagtta cctctgagga tctcaagcgc    1920
   agctatgtct gtcatgctag aagtgccaaa ggcgaagttg ccaaagcagc caaggtgaag    1980
   cagaaagtgc cagctccaag atacacagtg gaatccggag agtccaaata cggtccgcca    2040
   tgcccatcat gcccagcacc tgagttcctg gggggaccat cagtcttcct gttcccccca    2100
   aaacccaagg acactctcat gatctcccgg acccctgagg tcacgtgcgt ggtggtggac    2160
   gtgagccagg aagaccccga ggtccagttc aactggtacg tggatggcgt ggaggtgcat    2220
   aatgccaaga caaagccgcg ggaggagcag ttcaacagca cgtaccgtgt ggtcagcgtc    2280
   ctcaccgtcc tgcaccagga ctggctgaac ggcaaggagt acaagtgcaa ggtctccaac    2340
   aaaggcctcc cgtcctccat cgagaaaacc atctccaaag ccaaagggca gccccgagag    2400
   ccacaggtgt acaccctgcc cccatcccag gaggagatga ccaagaacca ggtcagcctg    2460
   acctgcctgg tcaaaggctt ctaccccagc gacatcgccg tggagtggga gagcaatggg    2520
   cagccggaga acaactacaa gaccacgcct cccgtgctgg actccgacgg ctccttcttc    2580
   ctctacagca ggctaaccgt ggacaagagc aggtggcagg aggggaatgt cttctcatgc    2640
   tccgtgatgc atgaggctct gcacaaccac tacacacaga agagcctctc cctgtctctg    2700
   ggtaaatga                                                            2709
<210> 6
   <211> 902
   <212> PRT
   <213> Homo sapiens
<400> 6
   Met Val Leu Leu Arg Leu Ile Cys Phe Ile Ala Leu Leu Ile Ser Ser
   1               5                   10                  15
   Leu Glu Ala Asp Lys Cys Lys Glu Arg Glu Glu Lys Ile Ile Leu Val
               20                  25                  30
   Ser Ser Ala Asn Glu Ile Asp Val Arg Pro Cys Pro Leu Asn Pro Asn
           35                  40                  45
   Glu His Lys Gly Thr Ile Thr Trp Tyr Lys Asp Asp Ser Lys Thr Pro
       50                  55                  60
   Val Ser Thr Glu Gln Ala Ser Arg Ile His Gln His Lys Glu Lys Leu
   65                  70                  75                  80
   Trp Phe Val Pro Ala Lys Val Glu Asp Ser Gly His Tyr Tyr Cys Val
                   85                  90                  95
   Val Arg Asn Ser Ser Tyr Cys Leu Arg Ile Lys Ile Ser Ala Lys Phe
               100                 105                 110
   Val Glu Asn Glu Pro Asn Leu Cys Tyr Asn Ala Gln Ala Ile Phe Lys
           115                 120                 125
   Gln Lys Leu Pro Val Ala Gly Asp Gly Gly Leu Val Cys Pro Tyr Met
       130                 135                 140
   Glu Phe Phe Lys Asn Glu Asn Asn Glu Leu Pro Lys Leu Gln Trp Tyr
   145                 150                 155                 160
   Lys Asp Cys Lys Pro Leu Leu Leu Asp Asn Ile His Phe Ser Gly Val
                   165                 170                 175
   Lys Asp Arg Leu Ile Val Met Asn Val Ala Glu Lys His Arg Gly Asn
               180                 185                 190
   Tyr Thr Cys His Ala Ser Tyr Thr Tyr Leu Gly Lys Gln Tyr Pro Ile
           195                 200                 205
   Thr Arg Val Ile Glu Phe Ile Thr Leu Glu Glu Asn Lys Pro Thr Arg
       210                 215                 220
   Pro Val Ile Val Ser Pro Ala Asn Glu Thr Met Glu Val Asp Leu Gly
   225                 230                 235                 240
   Ser Gln Ile Gln Leu Ile Cys Asn Val Thr Gly Gln Leu Ser Asp Ile
                   245                 250                 255
   Ala Tyr Trp Lys Trp Asn Gly Ser Val Ile Asp Glu Asp Asp Pro Val
               260                 265                 270
   Leu Gly Glu Asp Tyr Tyr Ser Val Glu Asn Pro Ala Asn Lys Arg Arg
           275                 280                 285
   Ser Thr Leu Ile Thr Val Leu Asn Ile Ser Glu Ile Glu Ser Arg Phe
       290                 295                 300
   Tyr Lys His Pro Phe Thr Cys Phe Ala Lys Asn Thr His Gly Ile Asp
   305                 310                 315                 320
   Ala Ala Tyr Ile Gln Leu Ile Tyr Pro Val Thr Asn Ser Glu Arg Cys
                   325                 330                 335
   Asp Asp Trp Gly Leu Asp Thr Met Arg Gln Ile Gln Val Phe Glu Asp
               340                 345                 350
   Glu Pro Ala Arg Ile Lys Cys Pro Leu Phe Glu His Phe Leu Lys Phe
           355                 360                 365
   Asn Tyr Ser Thr Ala His Ser Ala Gly Leu Thr Leu Ile Trp Tyr Trp
       370                 375                 380
   Thr Arg Gln Asp Arg Asp Leu Glu Glu Pro Ile Asn Phe Arg Leu Pro
   385 390 395 400
   Glu Asn Arg Ile Ser Lys Glu Lys Asp Val Leu Trp Phe Arg Pro Thr
   405 410 415
   Leu Leu Asn Asp Thr Gly Asn Tyr Thr Cys Met Leu Arg Asn Thr Thr
   420 425 430
   Tyr Cys Ser Lys Val Ala Phe Pro Leu Glu Val Val Gln Lys Asp Ser
   435 440 445
   Cys Phe Asn Ser Pro Met Lys Leu Pro Val His Lys Leu Tyr Ile Glu
   450 455 460
   Tyr Gly Ile Gln Arg Ile Thr Cys Pro Asn Val Asp Gly Tyr Phe Pro
   465 470 475 480
   Ser Ser Val Lys Pro Thr Ile Thr Trp Tyr Met Gly Cys Tyr Lys Ile
   485 490 495
   Gln Asn Phe Asn Asn Val Ile Pro Glu Gly Met Asn Leu Ser Phe Leu
   500 505 510
   Ile Ala Leu Ile Ser Asn Asn Gly Asn Tyr Thr Cys Val Val Thr Tyr
   515 520 525
   Pro Glu Asn Gly Arg Thr Phe His Leu Thr Arg Thr Leu Thr Val Lys
   530 535 540
   Val Val Gly Ser Pro Lys Asn Ala Val Pro Pro Val Ile His Ser Pro
   545 550 555 560
   Asn Asp His Val Val Tyr Glu Lys Glu Pro Gly Glu Glu Leu Leu Ile
   565 570 575
   Pro Cys Thr Val Tyr Phe Ser Phe Leu Met Asp Ser Arg Asn Glu Val
   580 585 590
   Trp Trp Thr Ile Asp Gly Lys Lys Pro Asp Asp Ile Thr Ile Asp Val
   595 600 605
   Thr Ile Asn Glu Ser Ile Ser His Ser Arg Thr Glu Asp Glu Thr Arg
   610 615 620
   Thr Gln Ile Leu Ser Ile Lys Lys Val Thr Ser Glu Asp Leu Lys Arg
   625 630 635 640
   Ser Tyr Val Cys His Ala Arg Ser Ala Lys Gly Glu Val Ala Lys Ala
   645 650 655
   Ala Lys Val Lys Gln Lys Val Pro Ala Pro Arg Tyr Thr Val Glu Ser
   660 665 670
   Gly Glu Ser Lys Tyr Gly Pro Pro Cys Pro Ser Cys Pro Ala Pro Glu
   675 680 685
   Phe Leu Gly Gly Pro Ser Val Phe Leu Phe Pro Pro Lys Pro Lys Asp
   690 695 700
   Thr Leu Met Ile Ser Arg Thr Pro Glu Val Thr Cys Val Val Val Asp
   705 710 715 720
   Val Ser Gln Glu Asp Pro Glu Val Gln Phe Asn Trp Tyr Val Asp Gly
   725 730 735
   Val Glu Val His Asn Ala Lys Thr Lys Pro Arg Glu Glu Gln Phe Asn
   740 745 750
   Ser Thr Tyr Arg Val Val Ser Val Leu Thr Val Leu His Gln Asp Trp
   755 760 765
   Leu Asn Gly Lys Glu Tyr Lys Cys Lys Val Ser Asn Lys Gly Leu Pro
   770 775 780
   Ser Ser Ile Glu Lys Thr Ile Ser Lys Ala Lys Gly Gln Pro Arg Glu
   785 790 795 800
   Pro Gln Val Tyr Thr Leu Pro Pro Ser Gln Glu Glu Met Thr Lys Asn
   805 810 815
   Gln Val Ser Leu Thr Cys Leu Val Lys Gly Phe Tyr Pro Ser Asp Ile
   820 825 830
   Ala Val Glu Trp Glu Ser Asn Gly Gln Pro Glu Asn Asn Tyr Lys Thr
   835 840 845
   Thr Pro Pro Val Leu Asp Ser Asp Gly Ser Phe Phe Leu Tyr Ser Arg
   850 855 860
   Leu Thr Val Asp Lys Ser Arg Trp Gln Glu Gly Asn Val Phe Ser Cys
   865 870 875 880
   Ser Val Met His Glu Ala Leu His Asn His Tyr Thr Gln Lys Ser Leu
   885 890 895
   Ser Leu Ser Leu Gly Lys
   900
<210> 7
   <211> 2709
   <212> DNA
   <213> Homo sapiens
<400> 7
   atggtgttac tcagacttat ttgtttcata gctctactga tttcttctct ggaggctgat      60
   aaatgcaagg aacgtgaaga aaaaataatt ttagtgtcat ctgcaaatga aattgatgtt     120
   cgtccctgtc ctcttaaccc aaatgaacac aaaggcacta taacttggta taaggatgac     180
   agcaagacac ctgtatctac agaacaagcc tccaggattc atcaacacaa agagaaactt     240
   tggtttgttc ctgctaaggt ggaggattca ggacattact attgcgtggt aagaaattca     300
   tcttactgcc tcagaattaa aataagtgca aaatttgtgg agaatgagcc taacttatgt     360
   tataatgcac aagccatatt taagcagaaa ctacccgttg caggagacgg aggacttgtg     420
   tgcccttata tggagttttt taaaaatgaa aataatgagt tacctaaatt acagtggtat     480
   aaggattgca aacctctact tcttgacaat atacacttta gtggagtcaa agataggctc     540
   atcgtgatga atgtggctga aaagcataga gggaactata cttgtcatgc atcctacaca     600
   tacttgggca agcaatatcc tattacccgg gtaatagaat ttattactct agaggaaaac     660
   aaacccacaa ggcctgtgat tgtgagccca gctaatgaga caatggaagt agacttggga     720
   tcccagatac aattgatctg taatgtcacc ggccagttga gtgacattgc ttactggaag     780
   tggaatgggt cagtaattga tgaagatgac ccagtgctag gggaagacta ttacagtgtg     840
   gaaaatcctg caaacaaaag aaggagtacc ctcatcacag tgcttaatat atcggaaatt     900
   gagagtagat tttataaaca tccatttacc tgttttgcca agaatacaca tggtatagat     960
   gcagcatata tccagttaat atatccagtc actaattcag aacgctgcga tgactgggga    1020
   ctagacacca tgaggcaaat ccaagtgttt gaagatgagc cagctcgcat caagtgccca    1080
   ctctttgaac acttcttgaa attcaactac agcacagccc attcagctgg ccttactctg    1140
   atctggtatt ggactaggca ggaccgggac cttgaggagc caattaactt ccgcctcccc    1200
   gagaaccgca ttagtaagga gaaagatgtg ctgtggttcc ggcccactct cctcaatgac    1260
   actggcaact atacctgcat gttaaggaac actacatatt gcagcaaagt tgcatttccc    1320
   ttggaagttg ttcaaaaaga cagctgtttc aattccccca tgaaactccc agtgcataaa    1380
   ctgtatatag aatatggcat tcagaggatc acttgtccaa atgtagatgg atattttcct    1440
   tccagtgtca aaccgactat cacttggtat atgggctgtt ataaaataca gaattttaat    1500
   aatgtaatac ccgaaggtat gaacttgagt ttcctcattg ccttaatttc aaataatgga    1560
   aattacacat gtgttgttac atatccagaa aatggacgta cgtttcatct caccaggact    1620
   ctgactgtaa aggtagtagg ctctccaaaa aatgcagtgc cccctgtgat ccattcacct    1680
   aatgatcatg tggtctatga gaaagaacca ggagaggagc tactcattcc ctgtacggtc    1740
   tattttagtt ttctgatgga ttctcgcaat gaggtttggt ggaccattga tggaaaaaaa    1800
   cctgatgaca tcactattga tgtcaccatt aacgaaagta taagtcatag tagaacagaa    1860
   gatgaaacaa gaactcagat tttgagcatc aagaaagtta cctctgagga tctcaagcgc    1920
   agctatgtct gtcatgctag aagtgccaaa ggcgaagttg ccaaagcagc caaggtgaag    1980
   cagaaagtgc cagctccaag atacacagtg gaatccggag agtccaaata cggtccgcca    2040
   tgcccaccat gcccagcacc tgagttcctg gggggaccat cagtcttcct gttcccccca    2100
   aaacccaagg acactctcat gatctcccgg acccctgagg tcacgtgcgt ggtggtggac    2160
   gtgagccagg aagaccccga ggtccagttc aactggtacg tggatggcgt ggaggtgcat    2220
   aatgccaaga caaagccgcg ggaggagcag ttcaacagca cgtaccgtgt ggtcagcgtc    2280
   ctcaccgtcc tgcaccagga ctggctgaac ggcaaggagt acaagtgcaa ggtctccaac    2340
   aaaggcctcc cgtcctccat cgagaaaacc atctccaaag ccaaagggca gccccgagag    2400
   ccacaggtgt acaccctgcc cccatcccag gaggagatga ccaagaacca ggtcagcctg    2460
   acctgcctgg tcaaaggctt ctaccccagc gacatcgccg tggagtggga gagcaatggg    2520
   cagccggaga acaactacaa gaccacgcct cccgtgctgg actccgacgg ctccttcttc    2580
   ctctacagca ggctaaccgt ggacaagagc aggtggcagg aggggaatgt cttctcatgc    2640
   tccgtgatgc atgaggctct gcacaaccac tacacacaga agagcctctc cctgtctctg    2700
   ggtaaatga                                                            2709
<210> 8
   <211> 902
   <212> PRT
   <213> Homo sapiens
<400> 8
<210> 9
   <211> 2703
   <212> DNA
   <213> Homo sapiens
<400> 9
   atggtgcttc tgtggtgtgt agtgagtctc tacttttatg gaatcctgca aagtgatgcc      60
   tcagaacgct gcgatgactg gggactagac accatgaggc aaatccaagt gtttgaagat     120
   gagccagctc gcatcaagtg cccactcttt gaacacttct tgaaattcaa ctacagcaca     180
   gcccattcag ctggccttac tctgatctgg tattggacta ggcaggaccg ggaccttgag     240
   gagccaatta acttccgcct ccccgagaac cgcattagta aggagaaaga tgtgctgtgg     300
   ttccggccca ctctcctcaa tgacactggc aactatacct gcatgttaag gaacactaca     360
   tattgcagca aagttgcatt tcccttggaa gttgttcaaa aagacagctg tttcaattcc     420
   cccatgaaac tcccagtgca taaactgtat atagaatatg gcattcagag gatcacttgt     480
   ccaaatgtag atggatattt tccttccagt gtcaaaccga ctatcacttg gtatatgggc     540
   tgttataaaa tacagaattt taataatgta atacccgaag gtatgaactt gagtttcctc     600
   attgccttaa tttcaaataa tggaaattac acatgtgttg ttacatatcc agaaaatgga     660
   cgtacgtttc atctcaccag gactctgact gtaaaggtag taggctctcc aaaaaatgca     720
   gtgccccctg tgatccattc acctaatgat catgtggtct atgagaaaga accaggagag     780
   gagctactca ttccctgtac ggtctatttt agttttctga tggattctcg caatgaggtt     840
   tggtggacca ttgatggaaa aaaacctgat gacatcacta ttgatgtcac cattaacgaa     900
   agtataagtc atagtagaac agaagatgaa acaagaactc agattttgag catcaagaaa     960
   gttacctctg aggatctcaa gcgcagctat gtctgtcatg ctagaagtgc caaaggcgaa    1020
   gttgccaaag cagccaaggt gaagcagaaa gtgccagctc caagatacac agtggaaaaa    1080
   tgcaaggaac gtgaagaaaa aataatttta gtgagctcag caaatgaaat cgatgttcgt    1140
   ccctgtcctc ttaacccaaa tgaacacaaa ggcactataa cttggtataa ggatgacagc    1200
   aagacacctg tatctacaga acaagcctcc aggattcatc aacacaaaga gaaactttgg    1260
   tttgttcctg ctaaggtgga ggattcagga cattactatt gcgtggtaag aaattcatct    1320
   tactgcctca gaattaaaat aagtgcaaaa tttgtggaga atgagcctaa cttatgttat    1380
   aatgcacaag ccatatttaa gcagaaacta cccgttgcag gagacggagg acttgtgtgc    1440
   ccttatatgg agttttttaa aaatgaaaat aatgagttac ctaaattaca gtggtataag    1500
   gattgcaaac ctctacttct tgacaatata cactttagtg gagtcaaaga taggctcatc    1560
   gtgatgaatg tggctgaaaa gcatagaggg aactatactt gtcatgcatc ctacacatac    1620
   ttgggcaagc aatatcctat tacccgggta atagaattta ttactctaga ggaaaacaaa    1680
   cccacaaggc ctgtgattgt gagcccagct aatgagacaa tggaagtaga cttgggatcc    1740
   cagatacaat tgatctgtaa tgtcaccggc cagttgagtg acattgctta ctggaagtgg    1800
   aatgggtcag taattgatga agatgaccca gtgctagggg aagactatta cagtgtggaa    1860
   aatcctgcaa acaaaagaag gagtaccctc atcacagtgc ttaatatatc ggaaattgag    1920
   agtagatttt ataaacatcc atttacctgt tttgccaaga atacacatgg tatagatgca    1980
   gcatatatcc agttaatata tccagtcact aattccggag acaaaactca cacatgccca    2040
   ccgtgcccag cacctgaact cctgggggga ccgtcagtct tcctcttccc cccaaaaccc    2100
   aaggacaccc tcatgatctc ccggacccct gaggtcacat gcgtggtggt ggacgtgagc    2160
   cacgaagacc ctgaggtcaa gttcaactgg tacgtggacg gcgtggaggt gcataatgcc    2220
   aagacaaagc cgcgggagga gcagtacaac agcacgtacc gtgtggtcag cgtcctcacc    2280
   gtcctgcacc aggactggct gaatggcaag gagtacaagt gcaaggtctc caacaaagcc    2340
   ctcccagccc ccatcgagaa aaccatctcc aaagccaaag ggcagccccg agaaccacag    2400
   gtgtacaccc tgcccccatc ccgggatgag ctgaccaaga accaggtcag cctgacctgc    2460
   ctggtcaaag gcttctatcc cagcgacatc gccgtggagt gggagagcaa tgggcagccg    2520
   gagaacaact acaagaccac gcctcccgtg ctggactccg acggctcctt cttcctctac    2580
   agcaagctca ccgtggacaa gagcaggtgg cagcagggga acgtcttctc atgctccgtg    2640
   atgcatgagg ctctgcacaa ccactacacg cagaagagcc tctccctgtc tccgggtaaa    2700
   tga                                                                  2703
<210> 10
   <211> 900
   <212> PRT
   <213> Homo sapiens
<400> 10
   Met Val Leu Leu Trp Cys Val Val Ser Leu Tyr Phe Tyr Gly Ile Leu
   1               5                   10                  15
   Gln Ser Asp Ala Ser Glu Arg Cys Asp Asp Trp Gly Leu Asp Thr Met
               20                  25                  30
   Arg Gln Ile Gln Val Phe Glu Asp Glu Pro Ala Arg Ile Lys Cys Pro
               35                  40                  45
   Leu Phe Glu His Phe Leu Lys Phe Asn Tyr Ser Thr Ala His Ser Ala
           50                  55                  60
   Gly Leu Thr Leu Ile Trp Tyr Trp Thr Arg Gln Asp Arg Asp Leu Glu
   65                  70                  75                  80
   Glu Pro Ile Asn Phe Arg Leu Pro Glu Asn Arg Ile Ser Lys Glu Lys
                   85                  90                  95
   Asp Val Leu Trp Phe Arg Pro Thr Leu Leu Asn Asp Thr Gly Asn Tyr
               100                 105                 110
   Thr Cys Met Leu Arg Asn Thr Thr Tyr Cys Ser Lys Val Ala Phe Pro
           115                 120                 125
   Leu Glu Val Val Gln Lys Asp Ser Cys Phe Asn Ser Pro Met Lys Leu
       130                 135                 140
   Pro Val His Lys Leu Tyr Ile Glu Tyr Gly Ile Gln Arg Ile Thr Cys
   145                 150                 155                 160
   Pro Asn Val Asp Gly Tyr Phe Pro Ser Ser Val Lys Pro Thr Ile Thr
                   165                 170                 175
   Trp Tyr Met Gly Cys Tyr Lys Ile Gln Asn Phe Asn Asn Val Ile Pro
               180                 185                 190
   Glu Gly Met Asn Leu Ser Phe Leu Ile Ala Leu Ile Ser Asn Asn Gly
           195                 200                 205
   Asn Tyr Thr Cys Val Val Thr Tyr Pro Glu Asn Gly Arg Thr Phe His
       210                 215                 220
   Leu Thr Arg Thr Leu Thr Val Lys Val Val Gly Ser Pro Lys Asn Ala
   225                 230                 235                 240
   Val Pro Pro Val Ile His Ser Pro Asn Asp His Val Val Tyr Glu Lys
                   245                 250                 255
   Glu Pro Gly Glu Glu Leu Leu Ile Pro Cys Thr Val Tyr Phe Ser Phe
               260                 265                 270
   Leu Met Asp Ser Arg Asn Glu Val Trp Trp Thr Ile Asp Gly Lys Lys
           275                 280                 285
   Pro Asp Asp Ile Thr Ile Asp Val Thr Ile Asn Glu Ser Ile Ser His
       290                 295                 300
   Ser Arg Thr Glu Asp Glu Thr Arg Thr Gln Ile Leu Ser Ile Lys Lys
   305                 310                 315                 320
   Val Thr Ser Glu Asp Leu Lys Arg Ser Tyr Val Cys His Ala Arg Ser
                   325                 330                 335
   Ala Lys Gly Glu Val Ala Lys Ala Ala Lys Val Lys Gln Lys Val Pro
               340                 345                 350
   Ala Pro Arg Tyr Thr Val Glu Lys Cys Lys Glu Arg Glu Glu Lys Ile
           355                 360                 365
   Ile Leu Val Ser Ser Ala Asn Glu Ile Asp Val Arg Pro Cys Pro Leu
       370                 375                 380
   Asn Pro Asn Glu His Lys Gly Thr Ile Thr Trp Tyr Lys Asp Asp Ser
   385                 390                 395                 400
   Lys Thr Pro Val Ser Thr Glu Gln Ala Ser Arg Ile His Gln His Lys
                   405                 410                 415
   Glu Lys Leu Trp Phe Val Pro Ala Lys Val Glu Asp Ser Gly His Tyr
               420                 425                 430
   Tyr Cys Val Val Arg Asn Ser Ser Tyr Cys Leu Arg Ile Lys Ile Ser
           435                 440                 445
   Ala Lys Phe Val Glu Asn Glu Pro Asn Leu Cys Tyr Asn Ala Gln Ala
       450                 455                 460
   Ile Phe Lys Gln Lys Leu Pro Val Ala Gly Asp Gly Gly Leu Val Cys
   465                 470                 475                 480
   Pro Tyr Met Glu Phe Phe Lys Asn Glu Asn Asn Glu Leu Pro Lys Leu
                   485                 490                 495
   Gln Trp Tyr Lys Asp Cys Lys Pro Leu Leu Leu Asp Asn Ile His Phe
               500                 505                 510
   Ser Gly Val Lys Asp Arg Leu Ile Val Met Asn Val Ala Glu Lys His
           515                 520                 525
   Arg Gly Asn Tyr Thr Cys His Ala Ser Tyr Thr Tyr Leu Gly Lys Gln
       530                 535                 540
   Tyr Pro Ile Thr Arg Val Ile Glu Phe Ile Thr Leu Glu Glu Asn Lys
   545                 550                 555                 560
   Pro Thr Arg Pro Val Ile Val Ser Pro Ala Asn Glu Thr Met Glu Val
                   565                 570                 575
   Asp Leu Gly Ser Gln Ile Gln Leu Ile Cys Asn Val Thr Gly Gln Leu
               580                 585                 590
   Ser Asp Ile Ala Tyr Trp Lys Trp Asn Gly Ser Val Ile Asp Glu Asp
           595                 600                 605
   Asp Pro Val Leu Gly Glu Asp Tyr Tyr Ser Val Glu Asn Pro Ala Asn
       610                 615                 620
   Lys Arg Arg Ser Thr Leu Ile Thr Val Leu Asn Ile Ser Glu Ile Glu
   625                 630                 635                 640
   Ser Arg Phe Tyr Lys His Pro Phe Thr Cys Phe Ala Lys Asn Thr His
                   645                 650                 655
   Gly Ile Asp Ala Ala Tyr Ile Gln Leu Ile Tyr Pro Val Thr Asn Ser
               660                 665                 670
   Gly Asp Lys Thr His Thr Cys Pro Pro Cys Pro Ala Pro Glu Leu Leu
           675                 680                 685
   Gly Gly Pro Ser Val Phe Leu Phe Pro Pro Lys Pro Lys Asp Thr Leu
       690                 695                 700
   Met Ile Ser Arg Thr Pro Glu Val Thr Cys Val Val Val Asp Val Ser
   705                 710                 715                 720
   His Glu Asp Pro Glu Val Lys Phe Asn Trp Tyr Val Asp Gly Val Glu
                   725                 730                 735
   Val His Asn Ala Lys Thr Lys Pro Arg Glu Glu Gln Tyr Asn Ser Thr
               740                 745                 750
   Tyr Arg Val Val Ser Val Leu Thr Val Leu His Gln Asp Trp Leu Asn
           755                 760                 765
   Gly Lys Glu Tyr Lys Cys Lys Val Ser Asn Lys Ala Leu Pro Ala Pro
       770                 775                 780
   Ile Glu Lys Thr Ile Ser Lys Ala Lys Gly Gln Pro Arg Glu Pro Gln
   785                 790                 795                 800
   Val Tyr Thr Leu Pro Pro Ser Arg Asp Glu Leu Thr Lys Asn Gln Val
                   805                 810                 815
   Ser Leu Thr Cys Leu Val Lys Gly Phe Tyr Pro Ser Asp Ile Ala Val
               820                 825                 830
   Glu Trp Glu Ser Asn Gly Gln Pro Glu Asn Asn Tyr Lys Thr Thr Pro
           835                 840                 845
   Pro Val Leu Asp Ser Asp Gly Ser Phe Phe Leu Tyr Ser Lys Leu Thr
       850                 855                 860
   Val Asp Lys Ser Arg Trp Gln Gln Gly Asn Val Phe Ser Cys Ser Val
   865                 870                 875                 880
   Met His Glu Ala Leu His Asn His Tyr Thr Gln Lys Ser Leu Ser Leu
                   885                 890                 895
   Ser Pro Gly Lys
               900
<210> 11
   <211> 2709
   <212> DNA
   <213> Homo sapiens
<400> 11
   atggtgcttc tgtggtgtgt agtgagtctc tacttttatg gaatcctgca aagtgatgcc      60
   tcagaacgct gcgatgactg gggactagac accatgaggc aaatccaagt gtttgaagat      120
   gagccagctc gcatcaagtg cccactcttt gaacacttct tgaaattcaa ctacagcaca     180
   gcccattcag ctggccttac tctgatctgg tattggacta ggcaggaccg ggaccttgag     240
   gagccaatta acttccgcct ccccgagaac cgcattagta aggagaaaga tgtgctgtgg     300
   ttccggccca ctctcctcaa tgacactggc aactatacct gcatgttaag gaacactaca     360
   tattgcagca aagttgcatt tcccttggaa gttgttcaaa aagacagctg tttcaattcc     420
   cccatgaaac tcccagtgca taaactgtat atagaatatg gcattcagag gatcacttgt     480
   ccaaatgtag atggatattt tccttccagt gtcaaaccga ctatcacttg gtatatgggc     540
   tgttataaaa tacagaattt taataatgta atacccgaag gtatgaactt gagtttcctc     600
   attgccttaa tttcaaataa tggaaattac acatgtgttg ttacatatcc agaaaatgga     660
   cgtacgtttc atctcaccag gactctgact gtaaaggtag taggctctcc aaaaaatgca     720
   gtgccccctg tgatccattc acctaatgat catgtggtct atgagaaaga accaggagag     780
   gagctactca ttccctgtac ggtctatttt agttttctga tggattctcg caatgaggtt     840
   tggtggacca ttgatggaaa aaaacctgat gacatcacta ttgatgtcac cattaacgaa     900
   agtataagtc atagtagaac agaagatgaa acaagaactc agattttgag catcaagaaa     960
   gttacctctg aggatctcaa gcgcagctat gtctgtcatg ctagaagtgc caaaggcgaa    1020
   gttgccaaag cagccaaggt gaagcagaaa gtgccagctc caagatacac agtggaaaaa    1080
   tgcaaggaac gtgaagaaaa aataatttta gtgagctcag caaatgaaat cgatgttcgt    1140
   ccctgtcctc ttaacccaaa tgaacacaaa ggcactataa cttggtataa ggatgacagc    1200
   aagacacctg tatctacaga acaagcctcc aggattcatc aacacaaaga gaaactttgg    1260
   tttgttcctg ctaaggtgga ggattcagga cattactatt gcgtggtaag aaattcatct    1320
   tactgcctca gaattaaaat aagtgcaaaa tttgtggaga atgagcctaa cttatgttat    1380
   aatgcacaag ccatatttaa gcagaaacta cccgttgcag gagacggagg acttgtgtgc    1440
   ccttatatgg agttttttaa aaatgaaaat aatgagttac ctaaattaca gtggtataag    1500
   gattgcaaac ctctacttct tgacaatata cactttagtg gagtcaaaga taggctcatc    1560
   gtgatgaatg tggctgaaaa gcatagaggg aactatactt gtcatgcatc ctacacatac    1620
   ttgggcaagc aatatcctat tacccgggta atagaattta ttactctaga ggaaaacaaa    1680
   cccacaaggc ctgtgattgt gagcccagct aatgagacaa tggaagtaga cttgggatcc    1740
   cagatacaat tgatctgtaa tgtcaccggc cagttgagtg acattgctta ctggaagtgg    1800
   aatgggtcag taattgatga agatgaccca gtgctagggg aagactatta cagtgtggaa    1860
   aatcctgcaa acaaaagaag gagtaccctc atcacagtgc ttaatatatc ggaaattgag    1920
   agtagatttt ataaacatcc atttacctgt tttgccaaga atacacatgg tatagatgca    1980
   gcatatatcc agttaatata tccagtcact aattccggag agtccaaata cggtccgcca    2040
   tgcccatcat gcccagcacc tgagttcctg gggggaccat cagtcttcct gttcccccca    2100
   aaacccaagg acactctcat gatctcccgg acccctgagg tcacgtgcgt ggtggtggac    2160
   gtgagccagg aagaccccga ggtccagttc aactggtacg tggatggcgt ggaggtgcat    2220
   aatgccaaga caaagccgcg ggaggagcag ttcaacagca cgtaccgtgt ggtcagcgtc    2280
   ctcaccgtcc tgcaccagga ctggctgaac ggcaaggagt acaagtgcaa ggtctccaac    2340
   aaaggcctcc cgtcctccat cgagaaaacc atctccaaag ccaaagggca gccccgagag    2400
   ccacaggtgt acaccctgcc cccatcccag gaggagatga ccaagaacca ggtcagcctg    2460
   acctgcctgg tcaaaggctt ctaccccagc gacatcgccg tggagtggga gagcaatggg    2520
   cagccggaga acaactacaa gaccacgcct cccgtgctgg actccgacgg ctccttcttc    2580
   ctctacagca ggctaaccgt ggacaagagc aggtggcagg aggggaatgt cttctcatgc    2640
   tccgtgatgc atgaggctct gcacaaccac tacacacaga agagcctctc cctgtctctg    2700
   ggtaaatga                                                           2709
<210> 12
   <211> 902
   <212> PRT
   <213> Homo sapiens
<400> 12
   Met Val Leu Leu Trp Cys Val Val Ser Leu Tyr Phe Tyr Gly Ile Leu
    1               5                   10                  15
   Gln Ser Asp Ala Ser Glu Arg Cys Asp Asp Trp Gly Leu Asp Thr Met
               20                  25                  30
   Arg Gln Ile Gln Val Phe Glu Asp Glu Pro Ala Arg Ile Lys Cys Pro
           35                  40                  45
   Leu Phe Glu His Phe Leu Lys Phe Asn Tyr Ser Thr Ala His Ser Ala
       50                  55                  60
   Gly Leu Thr Leu Ile Trp Tyr Trp Thr Arg Gln Asp Arg Asp Leu Glu
   65                  70                  75                  80
   Glu Pro Ile Asn Phe Arg Leu Pro Glu Asn Arg Ile Ser Lys Glu Lys
                   85                  90                  95
   Asp Val Leu Trp Phe Arg Pro Thr Leu Leu Asn Asp Thr Gly Asn Tyr
               100                 105                 110
   Thr Cys Met Leu Arg Asn Thr Thr Tyr Cys Ser Lys Val Ala Phe Pro
   115 120 125
   Leu Glu Val Val Gln Lys Asp Ser Cys Phe Asn Ser Pro Met Lys Leu
   130 135 140
   Pro Val His Lys Leu Tyr Ile Glu Tyr Gly Ile Gln Arg Ile Thr Cys
   145 150 155 160
   Pro Asn Val Asp Gly Tyr Phe Pro Ser Ser Val Lys Pro Thr Ile Thr
   165 170 175
   Trp Tyr Met Gly Cys Tyr Lys Ile Gln Asn Phe Asn Asn Val Ile Pro
   180 185 190
   Glu Gly Met Asn Leu Ser Phe Leu Ile Ala Leu Ile Ser Asn Asn Gly
   195 200 205
   Asn Tyr Thr Cys Val Val Thr Tyr Pro Glu Asn Gly Arg Thr Phe His
   210 215 220
   Leu Thr Arg Thr Leu Thr Val Lys Val Val Gly Ser Pro Lys Asn Ala
   225 230 235 240
   Val Pro Pro Val Ile His Ser Pro Asn Asp His Val Val Tyr Glu Lys
   245 250 255
   Glu Pro Gly Glu Glu Leu Leu Ile Pro Cys Thr Val Tyr Phe Ser Phe
   260 265 270
   Leu Met Asp Ser Arg Asn Glu Val Trp Trp Thr Ile Asp Gly Lys Lys
   275 280 285
   Pro Asp Asp Ile Thr Ile Asp Val Thr Ile Asn Glu Ser Ile Ser His
   290 295 300
   Ser Arg Thr Glu Asp Glu Thr Arg Thr Gln Ile Leu Ser Ile Lys Lys
   305 310 315 320
   Val Thr Ser Glu Asp Leu Lys Arg Ser Tyr Val Cys His Ala Arg Ser
   325 330 335
   Ala Lys Gly Glu Val Ala Lys Ala Ala Lys Val Lys Gln Lys Val Pro
   340 345 350
   Ala Pro Arg Tyr Thr Val Glu Lys Cys Lys Glu Arg Glu Glu Lys Ile
   355 360 365
   Ile Leu Val Ser Ser Ala Asn Glu Ile Asp Val Arg Pro Cys Pro Leu
   370 375 380
   Asn Pro Asn Glu His Lys Gly Thr Ile Thr Trp Tyr Lys Asp Asp Ser
   385 390 395 400
   Lys Thr Pro Val Ser Thr Glu Gln Ala Ser Arg Ile His Gln His Lys
   405 410 415
   Glu Lys Leu Trp Phe Val Pro Ala Lys Val Glu Asp Ser Gly His Tyr
   420 425 430
   Tyr Cys Val Val Arg Asn Ser Ser Tyr Cys Leu Arg Ile Lys Ile Ser
   435 440 445
   Ala Lys Phe Val Glu Asn Glu Pro Asn Leu Cys Tyr Asn Ala Gln Ala
   450 455 460
   Ile Phe Lys Gln Lys Leu Pro Val Ala Gly Asp Gly Gly Leu Val Cys
   465 470 475 480
   Pro Tyr Met Glu Phe Phe Lys Asn Glu Asn Asn Glu Leu Pro Lys Leu
   485 490 495
   Gln Trp Tyr Lys Asp Cys Lys Pro Leu Leu Leu Asp Asn Ile His Phe
   500 505 510
   Ser Gly Val Lys Asp Arg Leu Ile Val Met Asn Val Ala Glu Lys His
   515 520 525
   Arg Gly Asn Tyr Thr Cys His Ala Ser Tyr Thr Tyr Leu Gly Lys Gln
   530 535 540
   Tyr Pro Ile Thr Arg Val Ile Glu Phe Ile Thr Leu Glu Glu Asn Lys
   545 550 555 560
   Pro Thr Arg Pro Val Ile Val Ser Pro Ala Asn Glu Thr Met Glu Val
   565 570 575
   Asp Leu Gly Ser Gln Ile Gln Leu Ile Cys Asn Val Thr Gly Gln Leu
   580 585 590
   Ser Asp Ile Ala Tyr Trp Lys Trp Asn Gly Ser Val Ile Asp Glu Asp
                   595                     600                     605
   Asp Pro Val Leu Gly Glu Asp Tyr Tyr Ser Val Glu Asn Pro Ala Asn
       610                     615                     620
   Lys Arg Arg Ser Thr Leu Ile Thr Val Leu Asn Ile Ser Glu Ile Glu
   625                     630                     635                     640
   Ser Arg Phe Tyr Lys His Pro Phe Thr Cys Phe Ala Lys Asn Thr His
                   645                     650                     655
   Gly Ile Asp Ala Ala Tyr Ile Gln Leu Ile Tyr Pro Val Thr Asn Ser
               660                     665                     670
   Gly Glu Ser Lys Tyr Gly Pro Pro Cys Pro Ser Cys Pro Ala Pro Glu
           675                     680                     685
   Phe Leu Gly Gly Pro Ser Val Phe Leu Phe Pro Pro Lys Pro Lys Asp
       690                     695                     700
   Thr Leu Met Ile Ser Arg Thr Pro Glu Val Thr Cys Val Val Val Asp
   705                     710                     715                     720
   Val Ser Gln Glu Asp Pro Glu Val Gln Phe Asn Trp Tyr Val Asp Gly
                   725                     730                     735
   Val Glu Val His Asn Ala Lys Thr Lys Pro Arg Glu Glu Gln Phe Asn
               740                     745                     750
   Ser Thr Tyr Arg Val Val Ser Val Leu Thr Val Leu His Gln Asp Trp
           755                     760                     765
   Leu Asn Gly Lys Glu Tyr Lys Cys Lys Val Ser Asn Lys Gly Leu Pro
       770                     775                     780
   Ser Ser Ile Glu Lys Thr Ile Ser Lys Ala Lys Gly Gln Pro Arg Glu
   785                     790                     795                     800
   Pro Gln Val Tyr Thr Leu Pro Pro Ser Gln Glu Glu Met Thr Lys Asn
                   805                     810                     815
   Gln Val Ser Leu Thr Cys Leu Val Lys Gly Phe Tyr Pro Ser Asp Ile
               820                     825                     830
   Ala Val Glu Trp Glu Ser Asn Gly Gln Pro Glu Asn Asn Tyr Lys Thr
           835                     840                     845
   Thr Pro Pro Val Leu Asp Ser Asp Gly Ser Phe Phe Leu Tyr Ser Arg
       850                     855                     860
   Leu Thr Val Asp Lys Ser Arg Trp Gln Glu Gly Asn Val Phe Ser Cys
   865                     870                     875                     880
   Ser Val Met His Glu Ala Leu His Asn His Tyr Thr Gln Lys Ser Leu
                   885                     890                     895
   Ser Leu Ser Leu Gly Lys
               900
<210> 13
   <211> 2709
   <212> DNA
   <213> Homo sapiens
<400> 13
   atggtgcttc tgtggtgtgt agtgagtctc tacttttatg gaatcctgca aagtgatgcc      60
   tcagaacgct gcgatgactg gggactagac accatgaggc aaatccaagt gtttgaagat     120
   gagccagctc gcatcaagtg cccactcttt gaacacttct tgaaattcaa ctacagcaca     180
   gcccattcag ctggccttac tctgatctgg tattggacta ggcaggaccg ggaccttgag     240
   gagccaatta acttccgcct ccccgagaac cgcattagta aggagaaaga tgtgctgtgg     300
   ttccggccca ctctcctcaa tgacactggc aactatacct gcatgttaag gaacactaca     360
   tattgcagca aagttgcatt tcccttggaa gttgttcaaa aagacagctg tttcaattcc     420
   cccatgaaac tcccagtgca taaactgtat atagaatatg gcattcagag gatcacttgt     480
   ccaaatgtag atggatattt tccttccagt gtcaaaccga ctatcacttg gtatatgggc     540
   tgttataaaa tacagaattt taataatgta atacccgaag gtatgaactt gagtttcctc     600
   attgccttaa tttcaaataa tggaaattac acatgtgttg ttacatatcc agaaaatgga     660
   cgtacgtttc atctcaccag gactctgact gtaaaggtag taggctctcc aaaaaatgca     720
   gtgccccctg tgatccattc acctaatgat catgtggtct atgagaaaga accaggagag     780
   gagctactca ttccctgtac ggtctatttt agttttctga tggattctcg caatgaggtt     840
   tggtggacca ttgatggaaa aaaacctgat gacatcacta ttgatgtcac cattaacgaa     900
   agtataagtc atagtagaac agaagatgaa acaagaactc agattttgag catcaagaaa      960
   gttacctctg aggatctcaa gcgcagctat gtctgtcatg ctagaagtgc caaaggcgaa     1020
   gttgccaaag cagccaaggt gaagcagaaa gtgccagctc caagatacac agtggaaaaa     1080
   tgcaaggaac gtgaagaaaa aataatttta gtgagctcag caaatgaaat cgatgttcgt     1140
   ccctgtcctc ttaacccaaa tgaacacaaa ggcactataa cttggtataa ggatgacagc     1200
   aagacacctg tatctacaga acaagcctcc aggattcatc aacacaaaga gaaactttgg     1260
   tttgttcctg ctaaggtgga ggattcagga cattactatt gcgtggtaag aaattcatct     1320
   tactgcctca gaattaaaat aagtgcaaaa tttgtggaga atgagcctaa cttatgttat     1380
   aatgcacaag ccatatttaa gcagaaacta cccgttgcag gagacggagg acttgtgtgc     1440
   ccttatatgg agttttttaa aaatgaaaat aatgagttac ctaaattaca gtggtataag     1500
   gattgcaaac ctctacttct tgacaatata cactttagtg gagtcaaaga taggctcatc     1560
   gtgatgaatg tggctgaaaa gcatagaggg aactatactt gtcatgcatc ctacacatac     1620
   ttgggcaagc aatatcctat tacccgggta atagaattta ttactctaga ggaaaacaaa     1680
   cccacaaggc ctgtgattgt gagcccagct aatgagacaa tggaagtaga cttgggatcc     1740
   cagatacaat tgatctgtaa tgtcaccggc cagttgagtg acattgctta ctggaagtgg     1800
   aatgggtcag taattgatga agatgaccca gtgctagggg aagactatta cagtgtggaa     1860
   aatcctgcaa acaaaagaag gagtaccctc atcacagtgc ttaatatatc ggaaattgag     1920
   agtagatttt ataaacatcc atttacctgt tttgccaaga atacacatgg tatagatgca     1980
   gcatatatcc agttaatata tccagtcact aattccggag agtccaaata cggtccgcca     2040
   tgcccaccat gcccagcacc tgagttcctg gggggaccat cagtcttcct gttcccccca     2100
   aaacccaagg acactctcat gatctcccgg acccctgagg tcacgtgcgt ggtggtggac     2160
   gtgagccagg aagaccccga ggtccagttc aactggtacg tggatggcgt ggaggtgcat     2220
   aatgccaaga caaagccgcg ggaggagcag ttcaacagca cgtaccgtgt ggtcagcgtc     2280
   ctcaccgtcc tgcaccagga ctggctgaac ggcaaggagt acaagtgcaa ggtctccaac     2340
   aaaggcctcc cgtcctccat cgagaaaacc atctccaaag ccaaagggca gccccgagag     2400
   ccacaggtgt acaccctgcc cccatcccag gaggagatga ccaagaacca ggtcagcctg     2460
   acctgcctgg tcaaaggctt ctaccccagc gacatcgccg tggagtggga gagcaatggg     2520
   cagccggaga acaactacaa gaccacgcct cccgtgctgg actccgacgg ctccttcttc     2580
   ctctacagca ggctaaccgt ggacaagagc aggtggcagg aggggaatgt cttctcatgc     2640
   tccgtgatgc atgaggctct gcacaaccac tacacacaga agagcctctc cctgtctctg     2700
   ggtaaatga                                                             2709
<210> 14
   <211> 902
   <212> PRT
   <213> Homo sapiens
<900> 14
<210> 15
   <211> 2748
   <212> DNA
   <213> Homo sapiens
<400> 15
   atggtgcgct tgtacgtgtt ggtaatggga gtttctgcct tcacccttca gcctgcggca     60
   cacacagggg ctgccagaag ctgccggttt cgtgggaggc attacaagcg ggagttcagg    120
   ctggaagggg agcctgtagc cctgaggtgc ccccaggtgc cctactggtt gtgggcctct    180
   gtcagccccc gcatcaacct gacatggcat aaaaatgact ctgctaggac ggtcccagga    240
   gaagaagaga cacggatgtg ggcccaggac ggtgctctgt ggcttctgcc agccttgcag    300
   gaggactctg gcacctacgt ctgcactact agaaatgctt cttactgtga caaaatgtcc    360
   attgagctca gagtttttga gaatacagat gctttcctgc cgttcatctc atacccgcaa    420
   attttaacct tgtcaacctc tggggtatta gtatgccctg acctgagtga attcacccgt    480
   gacaaaactg acgtgaagat tcaatggtac aaggattctc ttcttttgga taaagacaat    540
   gagaaatttc taagtgtgag ggggaccact cacttactcg tacacgatgt ggccctggaa    600
   gatgctggct attaccgctg tgtcctgaca tttgcccatg aaggccagca atacaacatc    660
   actaggagta ttgagctacg catcaagaaa aaaaaagaag agaccattcc tgtgatcatt    720
   tcccccctca agaccatatc agcttctctg gggtcaagac tgacaatccc atgtaaggtg    780
   tttctgggaa ccggcacacc cttaaccacc atgctgtggt ggacggccaa tgacacccac    840
   atagagagcg cctacccggg aggccgcgtg accgaggggc cacgccagga atattcagaa    900
   aataatgaga actacattga agtgccattg atttttgatc ctgtcacaag agaggatttg    960
   cacatggatt ttaaatgtgt tgtccataat accctgagtt ttcagacact acgcaccaca   1020
   gtcaaggaag cctcctccac gttctcagaa cgctgcgatg actggggact agacaccatg   1080
   aggcaaatcc aagtgtttga agatgagcca gctcgcatca agtgcccact ctttgaacac   1140
   ttcttgaaat tcaactacag cacagcccat tcagctggcc ttactctgat ctggtattgg   1200
   actaggcagg accgggacct tgaggagcca attaacttcc gcctccccga gaaccgcatt   1260
   agtaaggaga aagatgtgct gtggttccgg cccactctcc tcaatgacac tggcaactat   1320
   acctgcatgt taaggaacac tacatattgc agcaaagttg catttccctt ggaagttgtt   1380
   caaaaagaca gctgtttcaa ttcccccatg aaactcccag tgcataaact gtatatagaa   1440
   tatggcattc agaggatcac ttgtccaaat gtagatggat attttccttc cagtgtcaaa     1500
   ccgactatca cttggtatat gggctgttat aaaatacaga attttaataa tgtaataccc     1560
   gaaggtatga acttgagttt cctcattgcc ttaatttcaa ataatggaaa ttacacatgt     1620
   gttgttacat atccagaaaa tggacgtacg tttcatctca ccaggactct gactgtaaag     1680
   gtagtaggct ctccaaaaaa tgcagtgccc cctgtgatcc attcacctaa tgatcatgtg     1740
   gtctatgaga aagaaccagg agaggagcta ctcattccct gtacggtcta ttttagtttt     1800
   ctgatggatt ctcgcaatga ggtttggtgg accattgatg gaaaaaaacc tgatgacatc     1860
   actattgatg tcaccattaa cgaaagtata agtcatagta gaacagaaga tgaaacaaga     1920
   actcagattt tgagcatcaa gaaagttacc tctgaggatc tcaagcgcag ctatgtctgt     1980
   catgctagaa gtgccaaagg cgaagttgcc aaagcagcca aggtgaagca gaaagtgcca     2040
   gctccaagat acacagtgtc cggagacaaa actcacacat gcccaccgtg cccagcacct     2100
   gaactcctgg ggggaccgtc agtcttcctc ttccccccaa aacccaagga caccctcatg     2160
   atctcccgga cccctgaggt cacatgcgtg gtggtggacg tgagccacga agaccctgag     2220
   gtcaagttca actggtacgt ggacggcgtg gaggtgcata atgccaagac aaagccgcgg     2280
   gaggagcagt acaacagcac gtaccgtgtg gtcagcgtcc tcaccgtcct gcaccaggac     2340
   tggctgaatg gcaaggagta caagtgcaag gtctccaaca aagccctccc agcccccatc     2400
   gagaaaacca tctccaaagc caaagggcag ccccgagaac cacaggtgta caccctgccc     2460
   ccatcccggg atgagctgac caagaaccag gtcagcctga cctgcctggt caaaggcttc     2520
   tatcccagcg acatcgccgt ggagtgggag agcaatgggc agccggagaa caactacaag     2580
   accacgcctc ccgtgctgga ctccgacggc tccttcttcc tctatagcaa gctcaccgtg     2640
   gacaagagca ggtggcagca ggggaacgtc ttctcatgct ccgtgatgca tgaggctctg     2700
   cacaaccact acacgcagaa gagcctctcc ctgtctccgg gtaaatga                 2748
<210> 16
   <211> 915
   <212> PRT
   <213> Homo sapiens
<400> 16
   Met Val Arg Leu Tyr Val Leu Val Met Gly Val Ser Ala Phe Thr Leu
   1               5                   10                  15
   Gln Pro Ala Ala His Thr Gly Ala Ala Arg Ser Cys Arg Phe Arg Gly
               20                  25                  30
   Arg His Tyr Lys Arg Glu Phe Arg Leu Glu Gly Glu Pro Val Ala Leu
           35                  40                  45
   Arg Cys Pro Gln Val Pro Tyr Trp Leu Trp Ala Ser Val Ser Pro Arg
       50                  55                  60
   Ile Asn Leu Thr Trp His Lys Asn Asp Ser Ala Arg Thr Val Pro Gly
   65                  70                  75                  80
   Glu Glu Glu Thr Arg Met Trp Ala Gln Asp Gly Ala Leu Trp Leu Leu
                   85                  90                  95
   Pro Ala Leu Gln Glu Asp Ser Gly Thr Tyr Val Cys Thr Thr Arg Asn
               100                 105                 110
   Ala Ser Tyr Cys Asp Lys Met Ser Ile Glu Leu Arg Val Phe Glu Asn
           115                 120                 125
   Thr Asp Ala Phe Leu Pro Phe Ile Ser Tyr Pro Gln Ile Leu Thr Leu
       130                 135                 140
   Ser Thr Ser Gly Val Leu Val Cys Pro Asp Leu Ser Glu Phe Thr Arg
   145                 150                 155                 160
   Asp Lys Thr Asp Val Lys Ile Gln Trp Tyr Lys Asp Ser Leu Leu Leu
                   165                 170                 175
   Asp Lys Asp Asn Glu Lys Phe Leu Ser Val Arg Gly Thr Thr His Leu
               180                 185                 190
   Leu Val His Asp Val Ala Leu Glu Asp Ala Gly Tyr Tyr Arg Cys Val
           195                 200                 205
   Leu Thr Phe Ala His Glu Gly Gln Gln Tyr Asn Ile Thr Arg Ser Ile
       210                 215                 220
   Glu Leu Arg Ile Lys Lys Lys Lys Glu Glu Thr Ile Pro Val Ile Ile
   225                 230                 235                 240
   Ser Pro Leu Lys Thr Ile Ser Ala Ser Leu Gly Ser Arg Leu Thr Ile
                   245                 250                 255
   Pro Cys Lys Val Phe Leu Gly Thr Gly Thr Pro Leu Thr Thr Met Leu
   260 265 270
   Trp Trp Thr Ala Asn Asp Thr His Ile Glu Ser Ala Tyr Pro Gly Gly
   275 280 285
   Arg Val Thr Glu Gly Pro Arg Gln Glu Tyr Ser Glu Asn Asn Glu Asn
   290 295 300
   Tyr Ile Glu Val Pro Leu Ile Phe Asp Pro Val Thr Arg Glu Asp Leu
   305 310 315 320
   His Met Asp Phe Lys Cys Val Val His Asn Thr Leu Ser Phe Gln Thr
   325 330 335
   Leu Arg Thr Thr Val Lys Glu Ala Ser Ser Thr Phe Ser Glu Arg Cys
   340 345 350
   Asp Asp Trp Gly Leu Asp Thr Met Arg Gln Ile Gln Val Phe Glu Asp
   355 360 365
   Glu Pro Ala Arg Ile Lys Cys Pro Leu Phe Glu His Phe Leu Lys Phe
   370 375 380
   Asn Tyr Ser Thr Ala His Ser Ala Gly Leu Thr Leu Ile Trp Tyr Trp
   385 390 395 400
   Thr Arg Gln Asp Arg Asp Leu Glu Glu Pro Ile Asn Phe Arg Leu Pro
   405 410 415
   Glu Asn Arg Ile Ser Lys Glu Lys Asp Val Leu Trp Phe Arg Pro Thr
   420 425 430
   Leu Leu Asn Asp Thr Gly Asn Tyr Thr Cys Met Leu Arg Asn Thr Thr
   435 440 445
   Tyr Cys Ser Lys Val Ala Phe Pro Leu Glu Val Val Gln Lys Asp Ser
   450 455 460
   Cys Phe Asn Ser Pro Met Lys Leu Pro Val His Lys Leu Tyr Ile Glu
   465 470 475 480
   Tyr Gly Ile Gln Arg Ile Thr Cys Pro Asn Val Asp Gly Tyr Phe Pro
   485 490 495
   Ser Ser Val Lys Pro Thr Ile Thr Trp Tyr Met Gly Cys Tyr Lys Ile
   500 505 510
   Gln Asn Phe Asn Asn Val Ile Pro Glu Gly Met Asn Leu Ser Phe Leu
   515 520 525
   Ile Ala Leu Ile Ser Asn Asn Gly Asn Tyr Thr Cys Val Val Thr Tyr
   530 535 540
   Pro Glu Asn Gly Arg Thr Phe His Leu Thr Arg Thr Leu Thr Val Lys
   545 550 555 560
   Val Val Gly Ser Pro Lys Asn Ala Val Pro Pro Val Ile His Ser Pro
   565 570 575
   Asn Asp His Val Val Tyr Glu Lys Glu Pro Gly Glu Glu Leu Leu Ile
   580 585 590
   Pro Cys Thr Val Tyr Phe Ser Phe Leu Met Asp Ser Arg Asn Glu Val
   595 600 605
   Trp Trp Thr Ile Asp Gly Lys Lys Pro Asp Asp Ile Thr Ile Asp Val
   610 615 620
   Thr Ile Asn Glu Ser Ile Ser His Ser Arg Thr Glu Asp Glu Thr Arg
   625 630 635 640
   Thr Gln Ile Leu Ser Ile Lys Lys Val Thr Ser Glu Asp Leu Lys Arg
   645 650 655
   Ser Tyr Val Cys His Ala Arg Ser Ala Lys Gly Glu Val Ala Lys Ala
   660 665 670
   Ala Lys Val Lys Gln Lys Val Pro Ala Pro Arg Tyr Thr Val Ser Gly
   675 680 685
   Asp Lys Thr His Thr Cys Pro Pro Cys Pro Ala Pro Glu Leu Leu Gly
   690 695 700
   Gly Pro Ser Val Phe Leu Phe Pro Pro Lys Pro Lys Asp Thr Leu Met
   705 710 715 720
   Ile Ser Arg Thr Pro Glu Val Thr Cys Val Val Val Asp Val Ser His
   725 730 735
   Glu Asp Pro Glu Val Lys Phe Asn Trp Tyr Val Asp Gly Val Glu Val
                   740                 745                 750
   His Asn Ala Lys Thr Lys Pro Arg Glu Glu Gln Tyr Asn Ser Thr Tyr
               755                 760                 765
   Arg Val Val Ser Val Leu Thr Val Leu His Gln Asp Trp Leu Asn Gly
           770                 775                 780
   Lys Glu Tyr Lys Cys Lys Val Ser Asn Lys Ala Leu Pro Ala Pro Ile
   785                 790                 795                 800
   Glu Lys Thr Ile Ser Lys Ala Lys Gly Gln Pro Arg Glu Pro Gln Val
                   805                 810                 815
   Tyr Thr Leu Pro Pro Ser Arg Asp Glu Leu Thr Lys Asn Gln Val Ser
                   820                 825                 830
   Leu Thr Cys Leu Val Lys Gly Phe Tyr Pro Ser Asp Ile Ala Val Glu
               835                 840                 845
   Trp Glu Ser Asn Gly Gln Pro Glu Asn Asn Tyr Lys Thr Thr Pro Pro
           850                 855                 860
   Val Leu Asp Ser Asp Gly Ser Phe Phe Leu Tyr Ser Lys Leu Thr Val
   865                 870                 875                 880
   Asp Lys Ser Arg Trp Gln Gln Gly Asn Val Phe Ser Cys Ser Val Met
                   885                 890                 895
   His Glu Ala Leu His Asn His Tyr Thr Gln Lys Ser Leu Ser Leu Ser
                   900                 905                 910
   Pro Gly Lys
               915
<210> 17
   <211> 2754
   <212> DNA
   <213> Homo sapiens
<400> 17
   atggtgcgct tgtacgtgtt ggtaatggga gtttctgcct tcacccttca gcctgcggca     60
   cacacagggg ctgccagaag ctgccggttt cgtgggaggc attacaagcg ggagttcagg    120
   ctggaagggg agcctgtagc cctgaggtgc ccccaggtgc cctactggtt gtgggcctct    180
   gtcagccccc gcatcaacct gacatggcat aaaaatgact ctgctaggac ggtcccagga    240
   gaagaagaga cacggatgtg ggcccaggac ggtgctctgt ggcttctgcc agccttgcag    300
   gaggactctg gcacctacgt ctgcactact agaaatgctt cttactgtga caaaatgtcc    360
   attgagctca gagtttttga gaatacagat gctttcctgc cgttcatctc atacccgcaa    420
   attttaacct tgtcaacctc tggggtatta gtatgccctg acctgagtga attcacccgt    480
   gacaaaactg acgtgaagat tcaatggtac aaggattctc ttcttttgga taaagacaat    540
   gagaaatttc taagtgtgag ggggaccact cacttactcg tacacgatgt ggccctggaa    600
   gatgctggct attaccgctg tgtcctgaca tttgcccatg aaggccagca atacaacatc    660
   actaggagta ttgagctacg catcaagaaa aaaaaagaag agaccattcc tgtgatcatt    720
   tcccccctca agaccatatc agcttctctg gggtcaagac tgacaatccc atgtaaggtg    780
   tttctgggaa ccggcacacc cttaaccacc atgctgtggt ggacggccaa tgacacccac    840
   atagagagcg cctacccggg aggccgcgtg accgaggggc cacgccagga atattcagaa    900
   aataatgaga actacattga agtgccattg atttttgatc ctgtcacaag agaggatttg    960
   cacatggatt ttaaatgtgt tgtccataat accctgagtt ttcagacact acgcaccaca   1020
   gtcaaggaag cctcctccac gttctcagaa cgctgcgatg actggggact agacaccatg   1080
   aggcaaatcc aagtgtttga agatgagcca gctcgcatca agtgcccact ctttgaacac   1140
   ttcttgaaat tcaactacag cacagcccat tcagctggcc ttactctgat ctggtattgg   1200
   actaggcagg accgggacct tgaggagcca attaacttcc gcctccccga gaaccgcatt   1260
   agtaaggaga aagatgtgct gtggttccgg cccactctcc tcaatgacac tggcaactat   1320
   acctgcatgt taaggaacac tacatattgc agcaaagttg catttccctt ggaagttgtt   1380
   caaaaagaca gctgtttcaa ttcccccatg aaactcccag tgcataaact gtatatagaa   1440
   tatggcattc agaggatcac ttgtccaaat gtagatggat attttccttc cagtgtcaaa   1500
   ccgactatca cttggtatat gggctgttat aaaatacaga attttaataa tgtaataccc    1560
   gaaggtatga acttgagttt cctcattgcc ttaatttcaa ataatggaaa ttacacatgt   1620
   gttgttacat atccagaaaa tggacgtacg tttcatctca ccaggactct gactgtaaag   1680
   gtagtaggct ctccaaaaaa tgcagtgccc cctgtgatcc attcacctaa tgatcatgtg   1740
   gtctatgaga aagaaccagg agaggagcta ctcattccct gtacggtcta ttttagtttt   1800
   ctgatggatt ctcgcaatga ggtttggtgg accattgatg gaaaaaaacc tgatgacatc   1860
   actattgatg tcaccattaa cgaaagtata agtcatagta gaacagaaga tgaaacaaga   1920
   actcagattt tgagcatcaa gaaagttacc tctgaggatc tcaagcgcag ctatgtctgt   1980
   catgctagaa gtgccaaagg cgaagttgcc aaagcagcca aggtgaagca gaaagtgcca   2040
   gctccaagat acacagtgtc cggagagtcc aaatacggtc cgccatgccc atcatgccca   2100
   gcacctgagt tcctgggggg accatcagtc ttcctgttcc ccccaaaacc caaggacact   2160
   ctcatgatct cccggacccc tgaggtcacg tgcgtggtgg tggacgtgag ccaggaagac   2220
   cccgaggtcc agttcaactg gtacgtggat ggcgtggagg tgcataatgc caagacaaag   2280
   ccgcgggagg agcagttcaa cagcacgtac cgtgtggtca gcgtcctcac cgtcctgcac   2340
   caggactggc tgaacggcaa ggagtacaag tgcaaggtct ccaacaaagg cctcccgtcc   2400
   tccatcgaga aaaccatctc caaagccaaa gggcagcccc gagagccaca ggtgtacacc   2460
   ctgcccccat cccaggagga gatgaccaag aaccaggtca gcctgacctg cctggtcaaa   2520
   ggcttctacc ccagcgacat cgccgtggag tgggagagca atgggcagcc ggagaacaac   2580
   tacaagacca cgcctcccgt gctggactcc gacggctcct tcttcctcta cagcaggcta   2640
   accgtggaca agagcaggtg gcaggagggg aatgtcttct catgctccgt gatgcatgag   2700
   gctctgcaca accactacac acagaagagc ctctccctgt ctctgggtaa atga         2754
<210> 18
   <211> 917
   <212> PRT
   <213> Homo sapiens
<400> 18
   Met Val Arg Leu Tyr Val Leu Val Met Gly Val Ser Ala Phe Thr Leu
   1               5                   10                  15
   Gln Pro Ala Ala His Thr Gly Ala Ala Arg Ser Cys Arg Phe Arg Gly
               20                  25                  30
   Arg His Tyr Lys Arg Glu Phe Arg Leu Glu Gly Glu Pro Val Ala Leu
           35                  40                  45
   Arg Cys Pro Gln Val Pro Tyr Trp Leu Trp Ala Ser Val Ser Pro Arg
       50                  55                  60
   Ile Asn Leu Thr Trp His Lys Asn Asp Ser Ala Arg Thr Val Pro Gly
   65                  70                  75                  80
   Glu Glu Glu Thr Arg Met Trp Ala Gln Asp Gly Ala Leu Trp Leu Leu
                   85                  90                  95
   Pro Ala Leu Gln Glu Asp Ser Gly Thr Tyr Val Cys Thr Thr Arg Asn
               100                 105                 110
   Ala Ser Tyr Cys Asp Lys Met Ser Ile Glu Leu Arg Val Phe Glu Asn
           115                 120                 125
   Thr Asp Ala Phe Leu Pro Phe Ile Ser Tyr Pro Gln Ile Leu Thr Leu
       130                 135                 140
   Ser Thr Ser Gly Val Leu Val Cys Pro Asp Leu Ser Glu Phe Thr Arg
   145                 150                 155                 160
   Asp Lys Thr Asp Val Lys Ile Gln Trp Tyr Lys Asp Ser Leu Leu Leu
                   165                 170                 175
   Asp Lys Asp Asn Glu Lys Phe Leu Ser Val Arg Gly Thr Thr His Leu
               180                 185                 190
   Leu Val His Asp Val Ala Leu Glu Asp Ala Gly Tyr Tyr Arg Cys Val
           195                 200                 205
   Leu Thr Phe Ala His Glu Gly Gln Gln Tyr Asn Ile Thr Arg Ser Ile
       210                 215                 220
   Glu Leu Arg Ile Lys Lys Lys Lys Glu Glu Thr Ile Pro Val Ile Ile
   225                 230                 235                 240
   Ser Pro Leu Lys Thr Ile Ser Ala Ser Leu Gly Ser Arg Leu Thr Ile
                   245                 250                 255
   Pro Cys Lys Val Phe Leu Gly Thr Gly Thr Pro Leu Thr Thr Met Leu
               260                 265                 270
   Trp Trp Thr Ala Asn Asp Thr His Ile Glu Ser Ala Tyr Pro Gly Gly
           275                 280                 285
   Arg Val Thr Glu Gly Pro Arg Gln Glu Tyr Ser Glu Asn Asn Glu Asn
       290                 295                 300
   Tyr Ile Glu Val Pro Leu Ile Phe Asp Pro Val Thr Arg Glu Asp Leu
   305                 310                 315                 320
   His Met Asp Phe Lys Cys Val Val His Asn Thr Leu Ser Phe Gln Thr
                   325                 330                 335
   Leu Arg Thr Thr Val Lys Glu Ala Ser Ser Thr Phe Ser Glu Arg Cys
               340                 345                 350
   Asp Asp Trp Gly Leu Asp Thr Met Arg Gln Ile Gln Val Phe Glu Asp
           355                 360                 365
   Glu Pro Ala Arg Ile Lys Cys Pro Leu Phe Glu His Phe Leu Lys Phe
       370                 375                 380
   Asn Tyr Ser Thr Ala His Ser Ala Gly Leu Thr Leu Ile Trp Tyr Trp
   385                 390                 395                 400
   Thr Arg Gln Asp Arg Asp Leu Glu Glu Pro Ile Asn Phe Arg Leu Pro
                   405                 410                 415
   Glu Asn Arg Ile Ser Lys Glu Lys Asp Val Leu Trp Phe Arg Pro Thr
               420                 425                 430
   Leu Leu Asn Asp Thr Gly Asn Tyr Thr Cys Met Leu Arg Asn Thr Thr
           435                 440                 445
   Tyr Cys Ser Lys Val Ala Phe Pro Leu Glu Val Val Gln Lys Asp Ser
       450                 455                 460
   Cys Phe Asn Ser Pro Met Lys Leu Pro Val His Lys Leu Tyr Ile Glu
   465                 470                 475                 480
   Tyr Gly Ile Gln Arg Ile Thr Cys Pro Asn Val Asp Gly Tyr Phe Pro
                   485                 490                 495
   Ser Ser Val Lys Pro Thr Ile Thr Trp Tyr Met Gly Cys Tyr Lys Ile
               500                 505                 510
   Gln Asn Phe Asn Asn Val Ile Pro Glu Gly Met Asn Leu Ser Phe Leu
           515                 520                 525
   Ile Ala Leu Ile Ser Asn Asn Gly Asn Tyr Thr Cys Val Val Thr Tyr
       530                 535                 540
   Pro Glu Asn Gly Arg Thr Phe His Leu Thr Arg Thr Leu Thr Val Lys
   545                 550                 555                 560
   Val Val Gly Ser Pro Lys Asn Ala Val Pro Pro Val Ile His Ser Pro
                   565                 570                 575
   Asn Asp His Val Val Tyr Glu Lys Glu Pro Gly Glu Glu Leu Leu Ile
               580                 585                 590
   Pro Cys Thr Val Tyr Phe Ser Phe Leu Met Asp Ser Arg Asn Glu Val
           595                 600                 605
   Trp Trp Thr Ile Asp Gly Lys Lys Pro Asp Asp Ile Thr Ile Asp Val
       610                 615                 620
   Thr Ile Asn Glu Ser Ile Ser His Ser Arg Thr Glu Asp Glu Thr Arg
   625                 630                 635                 640
   Thr Gln Ile Leu Ser Ile Lys Lys Val Thr Ser Glu Asp Leu Lys Arg
                   645                 650                 655
   Ser Tyr Val Cys His Ala Arg Ser Ala Lys Gly Glu Val Ala Lys Ala
               660                 665                 670
   Ala Lys Val Lys Gln Lys Val Pro Ala Pro Arg Tyr Thr Val Ser Gly
           675                 680                 685
   Glu Ser Lys Tyr Gly Pro Pro Cys Pro Ser Cys Pro Ala Pro Glu Phe
       690                 695                 700
   Leu Gly Gly Pro Ser Val Phe Leu Phe Pro Pro Lys Pro Lys Asp Thr
   705                 710                 715                 720
   Leu Met Ile Ser Arg Thr Pro Glu Val Thr Cys Val Val Val Asp Val
                   725                 730                 735
   Ser Gln Glu Asp Pro Glu Val Gln Phe Asn Trp Tyr Val Asp Gly Val
               740                 745                 750
   Glu Val His Asn Ala Lys Thr Lys Pro Arg Glu Glu Gln Phe Asn Ser
           755                 760                 765
   Thr Tyr Arg Val Val Ser Val Leu Thr Val Leu His Gln Asp Trp Leu
       770                 775                 780
   Asn Gly Lys Glu Tyr Lys Cys Lys Val Ser Asn Lys Gly Leu Pro Ser
   785                 790                 795                 800
   Ser Ile Glu Lys Thr Ile Ser Lys Ala Lys Gly Gln Pro Arg Glu Pro
   805 810 815
   Gln Val Tyr Thr Leu Pro Pro Ser Gln Glu Glu Met Thr Lys Asn Gln
   820 825 830
   Val Ser Leu Thr Cys Leu Val Lys Gly Phe Tyr Pro Ser Asp Ile Ala
   835 840 845
   Val Glu Trp Glu Ser Asn Gly Gln Pro Glu Asn Asn Tyr Lys Thr Thr
   850 855 860
   Pro Pro Val Leu Asp Ser Asp Gly Ser Phe Phe Leu Tyr Ser Arg Leu
   865 870 875 880
   Thr Val Asp Lys Ser Arg Trp Gln Glu Gly Asn Val Phe Ser Cys Ser
   885 890 895
   Val Met His Glu Ala Leu His Asn His Tyr Thr Gln Lys Ser Leu Ser
   900 905 910
   Leu Ser Leu Gly Lys
   915
<210> 19
   <211> 2754
   <212> DNA
   <213> Homo sapiens
<400> 19
   atggtgcgct tgtacgtgtt ggtaatggga gtttctgcct tcacccttca gcctgcggca 60
   cacacagggg ctgccagaag ctgccggttt cgtgggaggc attacaagcg ggagttcagg 120
   ctggaagggg agcctgtagc cctgaggtgc ccccaggtgc cctactggtt gtgggcctct 180
   gtcagccccc gcatcaacct gacatggcat aaaaatgact ctgctaggac ggtcccagga 240
   gaagaagaga cacggatgtg ggcccaggac ggtgctctgt ggcttctgcc agccttgcag 300
   gaggactctg gcacctacgt ctgcactact agaaatgctt cttactgtga caaaatgtcc 360
   attgagctca gagtttttga gaatacagat gctttcctgc cgttcatctc atacccgcaa 420
   attttaacct tgtcaacctc tggggtatta gtatgccctg acctgagtga attcacccgt 480
   gacaaaactg acgtgaagat tcaatggtac aaggattctc ttcttttgga taaagacaat 540
   gagaaatttc taagtgtgag ggggaccact cacttactcg tacacgatgt ggccctggaa 600
   gatgctggct attaccgctg tgtcctgaca tttgcccatg aaggccagca atacaacatc 660
   actaggagta ttgagctacg catcaagaaa aaaaaagaag agaccattcc tgtgatcatt 720
   tcccccctca agaccatatc agcttctctg gggtcaagac tgacaatccc atgtaaggtg 780
   tttctgggaa ccggcacacc cttaaccacc atgctgtggt ggacggccaa tgacacccac 840
   atagagagcg cctacccggg aggccgcgtg accgaggggc cacgccagga atattcagaa 900
   aataatgaga actacattga agtgccattg atttttgatc ctgtcacaag agaggatttg 960
   cacatggatt ttaaatgtgt tgtccataat accctgagtt ttcagacact acgcaccaca 1020
   gtcaaggaag cctcctccac gttctcagaa cgctgcgatg actggggact agacaccatg 1080
   aggcaaatcc aagtgtttga agatgagcca gctcgcatca agtgcccact ctttgaacac 1140
   ttcttgaaat tcaactacag cacagcccat tcagctggcc ttactctgat ctggtattgg 1200
   actaggcagg accgggacct tgaggagcca attaacttcc gcctccccga gaaccgcatt 1260
   agtaaggaga aagatgtgct gtggttccgg cccactctcc tcaatgacac tggcaactat 1320
   acctgcatgt taaggaacac tacatattgc agcaaagttg catttccctt ggaagttgtt 1380
   caaaaagaca gctgtttcaa ttcccccatg aaactcccag tgcataaact gtatatagaa 1440
   tatggcattc agaggatcac ttgtccaaat gtagatggat attttccttc cagtgtcaaa 1500
   ccgactatca cttggtatat gggctgttat aaaatacaga attttaataa tgtaataccc 1560
   gaaggtatga acttgagttt cctcattgcc ttaatttcaa ataatggaaa ttacacatgt 1620
   gttgttacat atccagaaaa tggacgtacg tttcatctca ccaggactct gactgtaaag 1680
   gtagtaggct ctccaaaaaa tgcagtgccc cctgtgatcc attcacctaa tgatcatgtg 1740
   gtctatgaga aagaaccagg agaggagcta ctcattccct gtacggtcta ttttagtttt 1800
   ctgatggatt ctcgcaatga ggtttggtgg accattgatg gaaaaaaacc tgatgacatc 1860
   actattgatg tcaccattaa cgaaagtata agtcatagta gaacagaaga tgaaacaaga 1920
   actcagattt tgagcatcaa gaaagttacc tctgaggatc tcaagcgcag ctatgtctgt 1980
   catgctagaa gtgccaaagg cgaagttgcc aaagcagcca aggtgaagca gaaagtgcca 2040
   gctccaagat acacagtgtc cggagagtcc aaatacggtc cgccatgccc accatgccca 2100
   gcacctgagt tcctgggggg accatcagtc ttcctgttcc ccccaaaacc caaggacact 2160
   ctcatgatct cccggacccc tgaggtcacg tgcgtggtgg tggacgtgag ccaggaagac 2220
   cccgaggtcc agttcaactg gtacgtggat ggcgtggagg tgcataatgc caagacaaag 2280
   ccgcgggagg agcagttcaa cagcacgtac cgtgtggtca gcgtcctcac cgtcctgcac    2340
   caggactggc tgaacggcaa ggagtacaag tgcaaggtct ccaacaaagg cctcccgtcc    2400
   tccatcgaga aaaccatctc caaagccaaa gggcagcccc gagagccaca ggtgtacacc    2460
   ctgcccccat cccaggagga gatgaccaag aaccaggtca gcctgacctg cctggtcaaa    2520
   ggcttctacc ccagcgacat cgccgtggag tgggagagca atgggcagcc ggagaacaac    2580
   tacaagacca cgcctcccgt gctggactcc gacggctcct tcttcctcta cagcaggcta    2640
   accgtggaca agagcaggtg gcaggagggg aatgtcttct catgctccgt gatgcatgag    2700
   gctctgcaca accactacac acagaagagc ctctccctgt ctctgggtaa atga          2754
<210> 20
   <211> 917
   <212> PRT
   <213> Homo sapiens
<400> 20
<210> 21
   <211> 2748
   <212> DNA
   <213> Homo sapiens
<400> 21
   atggtgcttc tgtggtgtgt agtgagtctc tacttttatg gaatcctgca aagtgatgcc      60
   tcagaacgct gcgatgactg gggactagac accatgaggc aaatccaagt gtttgaagat     120
   gagccagctc gcatcaagtg cccactcttt gaacacttct tgaaattcaa ctacagcaca     180
   gcccattcag ctggccttac tctgatctgg tattggacta ggcaggaccg ggaccttgag     240
   gagccaatta acttccgcct ccccgagaac cgcattagta aggagaaaga tgtgctgtgg     300
   ttccggccca ctctcctcaa tgacactggc aactatacct gcatgttaag gaacactaca     360
   tattgcagca aagttgcatt tcccttggaa gttgttcaaa aagacagctg tttcaattcc     420
   cccatgaaac tcccagtgca taaactgtat atagaatatg gcattcagag gatcacttgt     480
   ccaaatgtag atggatattt tccttccagt gtcaaaccga ctatcacttg gtatatgggc     540
   tgttataaaa tacagaattt taataatgta atacccgaag gtatgaactt gagtttcctc     600
   attgccttaa tttcaaataa tggaaattac acatgtgttg ttacatatcc agaaaatgga     660
   cgtacgtttc atctcaccag gactctgact gtaaaggtag taggctctcc aaaaaatgca     720
   gtgccccctg tgatccattc acctaatgat catgtggtct atgagaaaga accaggagag     780
   gagctactca ttccctgtac ggtctatttt agttttctga tggattctcg caatgaggtt    840
   tggtggacca ttgatggaaa aaaacctgat gacatcacta ttgatgtcac cattaacgaa     900
   agtataagtc atagtagaac agaagatgaa acaagaactc agattttgag catcaagaaa     960
   gttacctctg aggatctcaa gcgcagctat gtctgtcatg ctagaagtgc caaaggcgaa    1020
   gttgccaaag cagccaaggt gaagcagaaa gtgccagctc caagatacac agtgcacaca    1080
   ggggctgcca gaagctgccg gtttcgtggg aggcattaca agcgggagtt caggctggaa    1140
   ggggagcctg tagccctgag gtgcccccag gtgccctact ggttgtgggc ctctgtcagc    1200
   ccccgcatca acctgacatg gcataaaaat gactctgcta ggacggtccc aggagaagaa    1260
   gagacacgga tgtgggccca ggacggtgct ctgtggcttc tgccagcctt gcaggaggac    1320
   tctggcacct acgtctgcac tactagaaat gcttcttact gtgacaaaat gtccattgag    1380
   ctcagagttt ttgagaatac agatgctttc ctgccgttca tctcataccc gcaaatttta    1440
   accttgtcaa cctctggggt attagtatgc cctgacctga gtgaattcac ccgtgacaaa    1500
   actgacgtga agattcaatg gtacaaggat tctcttcttt tggataaaga caatgagaaa    1560
   tttctaagtg tgagggggac cactcactta ctcgtacacg atgtggccct ggaagatgct   1620
   ggctattacc gctgtgtcct gacatttgcc catgaaggcc agcaatacaa catcactagg    1680
   agtattgagc tacgcatcaa gaaaaaaaaa gaagagacca ttcctgtgat catttccccc    1740
   ctcaagacca tatcagcttc tctggggtca agactgacaa tcccatgtaa ggtgtttctg    1800
   ggaaccggca cacccttaac caccatgctg tggtggacgg ccaatgacac ccacatagag   1860
   agcgcctacc cgggaggccg cgtgaccgag ggccacgccc aggaatattc agaaaataat     1920
   gagaactaca ttgaagtgcc attgattttt gatcctgtca caagagagga tttgcacatg    1980
   gattttaaat gtgttgtcca taataccctg agttttcaga cactacgcac cacagtcaag    2040
   gaagcctcct ccacgttctc cggagacaaa actcacacat gcccaccgtg cccagcacct    2100
   gaactcctgg ggggaccgtc agtcttcctc ttccccccaa aacccaagga caccctcatg   2160
   atctcccgga cccctgaggt cacatgcgtg gtggtggacg tgagccacga agaccctgag    2220
   gtcaagttca actggtacgt ggacggcgtg gaggtgcata atgccaagac aaagccgcgg    2280
   gaggagcagt acaacagcac gtaccgtgtg gtcagcgtcc tcaccgtcct gcaccaggac    2340
   tggctgaatg gcaaggagta caagtgcaag gtctccaaca aagccctccc agcccccatc    2400
   gagaaaacca tctccaaagc caaagggcag ccccgagaac cacaggtgta caccctgccc    2460
   ccatcccggg atgagctgac caagaaccag gtcagcctga cctgcctggt caaaggcttc    2520
   tatcccagcg acatcgccgt ggagtgggag agcaatgggc agccggagaa caactacaag    2580
   accacgcctc ccgtgctgga ctccgacggc tccttcttcc tctatagcaa gctcaccgtg    2640
   gacaagagca ggtggcagca ggggaacgtc ttctcatgct ccgtgatgca tgaggctctg    2700
   cacaaccact acacgcagaa gagcctctcc ctgtctccgg gtaaatga                  2748
<210> 22
   <211> 915
   <212> PRT
   <213> Homo sapiens
<400> 22
   Met Val Leu Leu Trp Cys Val Val Ser Leu Tyr Phe Tyr Gly Ile Leu
   1               5                   10                  15
   Gln Ser Asp Ala Ser Glu Arg Cys Asp Asp Trp Gly Leu Asp Thr Met
               20                  25                  30
   Arg Gln Ile Gln Val Phe Glu Asp Glu Pro Ala Arg Ile Lys Cys Pro
           35                  40                  45
   Leu Phe Glu His Phe Leu Lys Phe Asn Tyr Ser Thr Ala His Ser Ala
       50                  55                  60
   Gly Leu Thr Leu Ile Trp Tyr Trp Thr Arg Gln Asp Arg Asp Leu Glu
   65                  70                  75                  80
   Glu Pro Ile Asn Phe Arg Leu Pro Glu Asn Arg Ile Ser Lys Glu Lys
                   85                  90                  95
   Asp Val Leu Trp Phe Arg Pro Thr Leu Leu Asn Asp Thr Gly Asn Tyr
               100                 105                 110
   Thr Cys Met Leu Arg Asn Thr Thr Tyr Cys Ser Lys Val Ala Phe Pro
           115                 120                 125
   Leu Glu Val Val Gln Lys Asp Ser Cys Phe Asn Ser Pro Met Lys Leu
       130                 135                 140
   Pro Val His Lys Leu Tyr Ile Glu Tyr Gly Ile Gln Arg Ile Thr Cys
   145                 150                 155                 160
   Pro Asn Val Asp Gly Tyr Phe Pro Ser Ser Val Lys Pro Thr Ile Thr
                   165                 170                 175
   Trp Tyr Met Gly Cys Tyr Lys Ile Gln Asn Phe Asn Asn Val Ile Pro
               180                 185                 190
   Glu Gly Met Asn Leu Ser Phe Leu Ile Ala Leu Ile Ser Asn Asn Gly
           195                 200                 205
   Asn Tyr Thr Cys Val Val Thr Tyr Pro Glu Asn Gly Arg Thr Phe His
       210                 215                 220
   Leu Thr Arg Thr Leu Thr Val Lys Val Val Gly Ser Pro Lys Asn Ala
   225                 230                 235                 240
   Val Pro Pro Val Ile His Ser Pro Asn Asp His Val Val Tyr Glu Lys
                   245                 250                 255
   Glu Pro Gly Glu Glu Leu Leu Ile Pro Cys Thr Val Tyr Phe Ser Phe
               260                 265                 270
   Leu Met Asp Ser Arg Asn Glu Val Trp Trp Thr Ile Asp Gly Lys Lys
           275                 280                 285
   Pro Asp Asp Ile Thr Ile Asp Val Thr Ile Asn Glu Ser Ile Ser His
       290                 295                 300
   Ser Arg Thr Glu Asp Glu Thr Arg Thr Gln Ile Leu Ser Ile Lys Lys
   305                 310                 315                 320
   Val Thr Ser Glu Asp Leu Lys Arg Ser Tyr Val Cys His Ala Arg Ser
                   325                 330                 335
   Ala Lys Gly Glu Val Ala Lys Ala Ala Lys Val Lys Gln Lys Val Pro
               340                 345                 350
   Ala Pro Arg Tyr Thr Val His Thr Gly Ala Ala Arg Ser Cys Arg Phe
           355                 360                 365
   Arg Gly Arg His Tyr Lys Arg Glu Phe Arg Leu Glu Gly Glu Pro Val
       370                 375                 380
   Ala Leu Arg Cys Pro Gln Val Pro Tyr Trp Leu Trp Ala Ser Val Ser
   385                 390                 395                 400
   Pro Arg Ile Asn Leu Thr Trp His Lys Asn Asp Ser Ala Arg Thr Val
                   405                 410                 415
   Pro Gly Glu Glu Glu Thr Arg Met Trp Ala Gln Asp Gly Ala Leu Trp
                   420                     425                     430
   Leu Leu Pro Ala Leu Gln Glu Asp Ser Gly Thr Tyr Val Cys Thr Thr
               435                     440                     445
   Arg Asn Ala Ser Tyr Cys Asp Lys Met Ser Ile Glu Leu Arg Val Phe
           450                     455                     460
   Glu Asn Thr Asp Ala Phe Leu Pro Phe Ile Ser Tyr Pro Gln Ile Leu
   465                     470                     475                     480
   Thr Leu Ser Thr Ser Gly Val Leu Val Cys Pro Asp Leu Ser Glu Phe
                       485                     490                     495
   Thr Arg Asp Lys Thr Asp Val Lys Ile Gln Trp Tyr Lys Asp Ser Leu
                   500                     505                     510
   Leu Leu Asp Lys Asp Asn Glu Lys Phe Leu Ser Val Arg Gly Thr Thr
               515                     520                     525
   His Leu Leu Val His Asp Val Ala Leu Glu Asp Ala Gly Tyr Tyr Arg
           530                     535                     540
   Cys Val Leu Thr Phe Ala His Glu Gly Gln Gln Tyr Asn Ile Thr Arg
   545                     550                     555                     560
   Ser Ile Glu Leu Arg Ile Lys Lys Lys Lys Glu Glu Thr Ile Pro Val
                       565                     570                     575
   Ile Ile Ser Pro Leu Lys Thr Ile Ser Ala Ser Leu Gly Ser Arg Leu
                   580                     585                     590
   Thr Ile Pro Cys Lys Val Phe Leu Gly Thr Gly Thr Pro Leu Thr Thr
               595                     600                     605
   Met Leu Trp Trp Thr Ala Asn Asp Thr His Ile Glu Ser Ala Tyr Pro
           610                     615                     620
   Gly Gly Arg Val Thr Glu Gly Pro Arg Gln Glu Tyr Ser Glu Asn Asn
   625                     630                     635                     640
   Glu Asn Tyr Ile Glu Val Pro Leu Ile Phe Asp Pro Val Thr Arg Glu
                       645                     650                     655
   Asp Leu His Met Asp Phe Lys Cys Val Val His Asn Thr Leu Ser Phe
                   660                     665                     670
   Gln Thr Leu Arg Thr Thr Val Lys Glu Ala Ser Ser Thr Phe Ser Gly
               675                     680                     685
   Asp Lys Thr His Thr Cys Pro Pro Cys Pro Ala Pro Glu Leu Leu Gly
           690                     695                     700
   Gly Pro Ser Val Phe Leu Phe Pro Pro Lys Pro Lys Asp Thr Leu Met
   705                     710                     715                     720
   Ile Ser Arg Thr Pro Glu Val Thr Cys Val Val Val Asp Val Ser His
                       725                     730                     735
   Glu Asp Pro Glu Val Lys Phe Asn Trp Tyr Val Asp Gly Val Glu Val
                   740                     745                     750
   His Asn Ala Lys Thr Lys Pro Arg Glu Glu Gln Tyr Asn Ser Thr Tyr
               755                     760                     765
   Arg Val Val Ser Val Leu Thr Val Leu His Gln Asp Trp Leu Asn Gly
           770                     775                     780
   Lys Glu Tyr Lys Cys Lys Val Ser Asn Lys Ala Leu Pro Ala Pro Ile
   785                     790                     795                     800
   Glu Lys Thr Ile Ser Lys Ala Lys Gly Gln Pro Arg Glu Pro Gln Val
                       805                     810                     815
   Tyr Thr Leu Pro Pro Ser Arg Asp Glu Leu Thr Lys Asn Gln Val Ser
                   820                     825                     830
   Leu Thr Cys Leu Val Lys Gly Phe Tyr Pro Ser Asp Ile Ala Val Glu
               835                     840                     845
   Trp Glu Ser Asn Gly Gln Pro Glu Asn Asn Tyr Lys Thr Thr Pro Pro
           850                     855                     860
   Val Leu Asp Ser Asp Gly Ser Phe Phe Leu Tyr Ser Lys Leu Thr Val
   865                     870                     875                     880
   Asp Lys Ser Arg Trp Gln Gln Gly Asn Val Phe Ser Cys Ser Val Met
                       885                     890                     895
   His Glu Ala Leu His Asn His Tyr Thr Gln Lys Ser Leu Ser Leu Ser
                   900                     905                     910
   Pro Gly Lys
           915
<210> 23
   <211> 2754
   <212> DNA
   <213> Homo sapiens
<400> 23
<210> 24
   <211> 917
   <212> PRT
   <213> Homo sapiens
<400> 24
   Met Val Leu Leu Trp Cys Val Val Ser Leu Tyr Phe Tyr Gly Ile Leu
   1               5                   10                  15
   Gln Ser Asp Ala Ser Glu Arg Cys Asp Asp Trp Gly Leu Asp Thr Met
               20                  25                  30
   Arg Gln Ile Gln Val Phe Glu Asp Glu Pro Ala Arg Ile Lys Cys Pro
           35                  40                  45
   Leu Phe Glu His Phe Leu Lys Phe Asn Tyr Ser Thr Ala His Ser Ala
       50                  55                  60
   Gly Leu Thr Leu Ile Trp Tyr Trp Thr Arg Gln Asp Arg Asp Leu Glu
   65                  70                  75                  80
   Glu Pro Ile Asn Phe Arg Leu Pro Glu Asn Arg Ile Ser Lys Glu Lys
                   85                  90                  95
   Asp Val Leu Trp Phe Arg Pro Thr Leu Leu Asn Asp Thr Gly Asn Tyr
               100                 105                 110
   Thr Cys Met Leu Arg Asn Thr Thr Tyr Cys Ser Lys Val Ala Phe Pro
           115                 120                 125
   Leu Glu Val Val Gln Lys Asp Ser Cys Phe Asn Ser Pro Met Lys Leu
       130                 135                 140
   Pro Val His Lys Leu Tyr Ile Glu Tyr Gly Ile Gln Arg Ile Thr Cys
   145                 150                 155                 160
   Pro Asn Val Asp Gly Tyr Phe Pro Ser Ser Val Lys Pro Thr Ile Thr
                   165                 170                 175
   Trp Tyr Met Gly Cys Tyr Lys Ile Gln Asn Phe Asn Asn Val Ile Pro
               180                 185                 190
   Glu Gly Met Asn Leu Ser Phe Leu Ile Ala Leu Ile Ser Asn Asn Gly
           195                 200                 205
   Asn Tyr Thr Cys Val Val Thr Tyr Pro Glu Asn Gly Arg Thr Phe His
       210                 215                 220
   Leu Thr Arg Thr Leu Thr Val Lys Val Val Gly Ser Pro Lys Asn Ala
   225                 230                 235                 240
   Val Pro Pro Val Ile His Ser Pro Asn Asp His Val Val Tyr Glu Lys
                   245                 250                 255
   Glu Pro Gly Glu Glu Leu Leu Ile Pro Cys Thr Val Tyr Phe Ser Phe
               260                 265                 270
   Leu Met Asp Ser Arg Asn Glu Val Trp Trp Thr Ile Asp Gly Lys Lys
           275                 280                 285
   Pro Asp Asp Ile Thr Ile Asp Val Thr Ile Asn Glu Ser Ile Ser His
       290                 295                 300
   Ser Arg Thr Glu Asp Glu Thr Arg Thr Gln Ile Leu Ser Ile Lys Lys
   305                 310                 315                 320
   Val Thr Ser Glu Asp Leu Lys Arg Ser Tyr Val Cys His Ala Arg Ser
                   325                 330                 335
   Ala Lys Gly Glu Val Ala Lys Ala Ala Lys Val Lys Gln Lys Val Pro
               340                 345                 350
   Ala Pro Arg Tyr Thr Val His Thr Gly Ala Ala Arg Ser Cys Arg Phe
           355                 360                 365
   Arg Gly Arg His Tyr Lys Arg Glu Phe Arg Leu Glu Gly Glu Pro Val
       370                 375                 380
   Ala Leu Arg Cys Pro Gln Val Pro Tyr Trp Leu Trp Ala Ser Val Ser
   385                 390                 395                 400
   Pro Arg Ile Asn Leu Thr Trp His Lys Asn Asp Ser Ala Arg Thr Val
                   405                 410                 415
   Pro Gly Glu Glu Glu Thr Arg Met Trp Ala Gln Asp Gly Ala Leu Trp
               420                 425                 430
   Leu Leu Pro Ala Leu Gln Glu Asp Ser Gly Thr Tyr Val Cys Thr Thr
           435                 440                 445
   Arg Asn Ala Ser Tyr Cys Asp Lys Met Ser Ile Glu Leu Arg Val Phe
       450                 455                 460
   Glu Asn Thr Asp Ala Phe Leu Pro Phe Ile Ser Tyr Pro Gln Ile Leu
   465                 470                 475                 480
   Thr Leu Ser Thr Ser Gly Val Leu Val Cys Pro Asp Leu Ser Glu Phe
                   485                 490                 495
   Thr Arg Asp Lys Thr Asp Val Lys Ile Gln Trp Tyr Lys Asp Ser Leu
               500                 505                 510
   Leu Leu Asp Lys Asp Asn Glu Lys Phe Leu Ser Val Arg Gly Thr Thr
           515                 520                 525
   His Leu Leu Val His Asp Val Ala Leu Glu Asp Ala Gly Tyr Tyr Arg
       530                 535                 540
   Cys Val Leu Thr Phe Ala His Glu Gly Gln Gln Tyr Asn Ile Thr Arg
   545                 550                 555                 560
   Ser Ile Glu Leu Arg Ile Lys Lys Lys Lys Glu Glu Thr Ile Pro Val
                   565                 570                 575
   Ile Ile Ser Pro Leu Lys Thr Ile Ser Ala Ser Leu Gly Ser Arg Leu
               580                 585                 590
   Thr Ile Pro Cys Lys Val Phe Leu Gly Thr Gly Thr Pro Leu Thr Thr
           595                 600                 605
   Met Leu Trp Trp Thr Ala Asn Asp Thr His Ile Glu Ser Ala Tyr Pro
       610                 615                 620
   Gly Gly Arg Val Thr Glu Gly Pro Arg Gln Glu Tyr Ser Glu Asn Asn
   625                 630                 635                 640
   Glu Asn Tyr Ile Glu Val Pro Leu Ile Phe Asp Pro Val Thr Arg Glu
                   645                 650                 655
   Asp Leu His Met Asp Phe Lys Cys Val Val His Asn Thr Leu Ser Phe
               660                 665                 670
   Gln Thr Leu Arg Thr Thr Val Lys Glu Ala Ser Ser Thr Phe Ser Gly
           675                 680                 685
   Glu Ser Lys Tyr Gly Pro Pro Cys Pro Ser Cys Pro Ala Pro Glu Phe
       690                 695                 700
   Leu Gly Gly Pro Ser Val Phe Leu Phe Pro Pro Lys Pro Lys Asp Thr
   705                 710                 715                 720
   Leu Met Ile Ser Arg Thr Pro Glu Val Thr Cys Val Val Val Asp Val
                   725                 730                 735
   Ser Gln Glu Asp Pro Glu Val Gln Phe Asn Trp Tyr Val Asp Gly Val
               740                 745                 750
   Glu Val His Asn Ala Lys Thr Lys Pro Arg Glu Glu Gln Phe Asn Ser
           755                 760                 765
   Thr Tyr Arg Val Val Ser Val Leu Thr Val Leu His Gln Asp Trp Leu
       770                 775                 780
   Asn Gly Lys Glu Tyr Lys Cys Lys Val Ser Asn Lys Gly Leu Pro Ser
   785                 790                 795                 800
   Ser Ile Glu Lys Thr Ile Ser Lys Ala Lys Gly Gln Pro Arg Glu Pro
                   805                 810                 815
   Gln Val Tyr Thr Leu Pro Pro Ser Gln Glu Glu Met Thr Lys Asn Gln
               820                 825                 830
   Val Ser Leu Thr Cys Leu Val Lys Gly Phe Tyr Pro Ser Asp Ile Ala
           835                 840                 845
   Val Glu Trp Glu Ser Asn Gly Gln Pro Glu Asn Asn Tyr Lys Thr Thr
       850                 855                 860
   Pro Pro Val Leu Asp Ser Asp Gly Ser Phe Phe Leu Tyr Ser Arg Leu
   865                 870                 875                 880
   Thr Val Asp Lys Ser Arg Trp Gln Glu Gly Asn Val Phe Ser Cys Ser
                   885                 890                 895
   Val Met His Glu Ala Leu His Asn His Tyr Thr Gln Lys Ser Leu Ser
               900                 905                 910
   Leu Ser Leu Gly Lys
           915
<210> 25
   <211> 2754
   <212> DNA
   <213> Homo sapiens
<400> 25
   atggtgcttc tgtggtgtgt agtgagtctc tacttttatg gaatcctgca aagtgatgcc      60
   tcagaacgct gcgatgactg gggactagac accatgaggc aaatccaagt gtttgaagat     120
   gagccagctc gcatcaagtg cccactcttt gaacacttct tgaaattcaa ctacagcaca     180
   gcccattcag ctggccttac tctgatctgg tattggacta ggcaggaccg ggaccttgag     240
   gagccaatta acttccgcct ccccgagaac cgcattagta aggagaaaga tgtgctgtgg     300
   ttccggccca ctctcctcaa tgacactggc aactatacct gcatgttaag gaacactaca     360
   tattgcagca aagttgcatt tcccttggaa gttgttcaaa aagacagctg tttcaattcc     420
   cccatgaaac tcccagtgca taaactgtat atagaatatg gcattcagag gatcacttgt     480
   ccaaatgtag atggatattt tccttccagt gtcaaaccga ctatcacttg gtatatgggc     540
   tgttataaaa tacagaattt taataatgta atacccgaag gtatgaactt gagtttcctc     600
   attgccttaa tttcaaataa tggaaattac acatgtgttg ttacatatcc agaaaatgga     660
   cgtacgtttc atctcaccag gactctgact gtaaaggtag taggctctcc aaaaaatgca     720
   gtgccccctg tgatccattc acctaatgat catgtggtct atgagaaaga accaggagag     780
   gagctactca ttccctgtac ggtctatttt agttttctga tggattctcg caatgaggtt     840
   tggtggacca ttgatggaaa aaaacctgat gacatcacta ttgatgtcac cattaacgaa     900
   agtataagtc atagtagaac agaagatgaa acaagaactc agattttgag catcaagaaa     960
   gttacctctg aggatctcaa gcgcagctat gtctgtcatg ctagaagtgc caaaggcgaa    1020
   gttgccaaag cagccaaggt gaagcagaaa gtgccagctc caagatacac agtgcacaca    1080
   ggggctgcca gaagctgccg gtttcgtggg aggcattaca agcgggagtt caggctggaa    1140
   ggggagcctg tagccctgag gtgcccccag gtgccctact ggttgtgggc ctctgtcagc    1200
   ccccgcatca acctgacatg gcataaaaat gactctgcta ggacggtccc aggagaagaa    1260
   gagacacgga tgtgggccca ggacggtgct ctgtggcttc tgccagcctt gcaggaggac    1320
   tctggcacct acgtctgcac tactagaaat gcttcttact gtgacaaaat gtccattgag    1380
   ctcagagttt ttgagaatac agatgctttc ctgccgttca tctcataccc gcaaatttta    1440
   accttgtcaa cctctggggt attagtatgc cctgacctga gtgaattcac ccgtgacaaa    1500
   actgacgtga agattcaatg gtacaaggat tctcttcttt tggataaaga caatgagaaa    1560
   tttctaagtg tgagggggac cactcactta ctcgtacacg atgtggccct ggaagatgct    1620
   ggctattacc gctgtgtcct gacatttgcc catgaaggcc agcaatacaa catcactagg    1680
   agtattgagc tacgcatcaa gaaaaaaaaa gaagagacca ttcctgtgat catttccccc    1740
   ctcaagacca tatcagcttc tctggggtca agactgacaa tcccatgtaa ggtgtttctg    1800
   ggaaccggca cacccttaac caccatgctg tggtggacgg ccaatgacac ccacatagag    1860
   agcgcctacc cgggaggccg cgtgaccgag gggccacgcc aggaatattc agaaaataat    1920
   gagaactaca ttgaagtgcc attgattttt gatcctgtca caagagagga tttgcacatg    1980
   gattttaaat gtgttgtcca taataccctg agttttcaga cactacgcac cacagtcaag    2040
   gaagcctcct ccacgttctc cggagagtcc aaatacggtc cgccatgccc accatgccca    2100
   gcacctgagt tcctgggggg accatcagtc ttcctgttcc ccccaaaacc caaggacact    2160
   ctcatgatct cccggacccc tgaggtcacg tgcgtggtgg tggacgtgag ccaggaagac    2220
   cccgaggtcc agttcaactg gtacgtggat ggcgtggagg tgcataatgc caagacaaag    2280
   ccgcgggagg agcagttcaa cagcacgtac cgtgtggtca gcgtcctcac cgtcctgcac    2340
   caggactggc tgaacggcaa ggagtacaag tgcaaggtct ccaacaaagg cctcccgtcc    2400
   tccatcgaga aaaccatctc caaagccaaa gggcagcccc gagagccaca ggtgtacacc    2460
   ctgcccccat cccaggagga gatgaccaag aaccaggtca gcctgacctg cctggtcaaa    2520
   ggcttctacc ccagcgacat cgccgtggag tgggagagca atgggcagcc ggagaacaac    2580
   tacaagacca cgcctcccgt gctggactcc gacggctcct tcttcctcta cagcaggcta    2640
   accgtggaca agagcaggtg gcaggagggg aatgtcttct catgctccgt gatgcatgag    2700
   gctctgcaca accactacac acagaagagc ctctccctgt ctctgggtaa atga          2754
<210> 26
   <211> 917
   <212> PRT
   <213> Homo sapiens
<400> 26
   Met Val Leu Leu Trp Cys Val Val Ser Leu Tyr Phe Tyr Gly Ile Leu
   1               5                   10                  15
   Gln Ser Asp Ala Ser Glu Arg Cys Asp Asp Trp Gly Leu Asp Thr Met
               20                  25                  30
   Arg Gln Ile Gln Val Phe Glu Asp Glu Pro Ala Arg Ile Lys Cys Pro
           35                  40                  45
   Leu Phe Glu His Phe Leu Lys Phe Asn Tyr Ser Thr Ala His Ser Ala
       50                  55                  60
   Gly Leu Thr Leu Ile Trp Tyr Trp Thr Arg Gln Asp Arg Asp Leu Glu
   65                  70                  75                  80
   Glu Pro Ile Asn Phe Arg Leu Pro Glu Asn Arg Ile Ser Lys Glu Lys
                   85                  90                  95
   Asp Val Leu Trp Phe Arg Pro Thr Leu Leu Asn Asp Thr Gly Asn Tyr
               100                 105                 110
   Thr Cys Met Leu Arg Asn Thr Thr Tyr Cys Ser Lys Val Ala Phe Pro
           115                 120                 125
   Leu Glu Val Val Gln Lys Asp Ser Cys Phe Asn Ser Pro Met Lys Leu
       130                 135                 140
   Pro Val His Lys Leu Tyr Ile Glu Tyr Gly Ile Gln Arg Ile Thr Cys
   145                 150                 155                 160
   Pro Asn Val Asp Gly Tyr Phe Pro Ser Ser Val Lys Pro Thr Ile Thr
                   165                 170                 175
   Trp Tyr Met Gly Cys Tyr Lys Ile Gln Asn Phe Asn Asn Val Ile Pro
               180                 185                 190
   Glu Gly Met Asn Leu Ser Phe Leu Ile Ala Leu Ile Ser Asn Asn Gly
           195                 200                 205
   Asn Tyr Thr Cys Val Val Thr Tyr Pro Glu Asn Gly Arg Thr Phe His
       210                 215                 220
   Leu Thr Arg Thr Leu Thr Val Lys Val Val Gly Ser Pro Lys Asn Ala
   225                 230                 235                 240
   Val Pro Pro Val Ile His Ser Pro Asn Asp His Val Val Tyr Glu Lys
                   245                 250                 255
   Glu Pro Gly Glu Glu Leu Leu Ile Pro Cys Thr Val Tyr Phe Ser Phe
               260                 265                 270
   Leu Met Asp Ser Arg Asn Glu Val Trp Trp Thr Ile Asp Gly Lys Lys
           275                 280                 285
   Pro Asp Asp Ile Thr Ile Asp Val Thr Ile Asn Glu Ser Ile Ser His
       290                 295                 300
   Ser Arg Thr Glu Asp Glu Thr Arg Thr Gln Ile Leu Ser Ile Lys Lys
   305                 310                 315                 320
   Val Thr Ser Glu Asp Leu Lys Arg Ser Tyr Val Cys His Ala Arg Ser
                   325                 330                 335
   Ala Lys Gly Glu Val Ala Lys Ala Ala Lys Val Lys Gln Lys Val Pro
               340                 345                 350
   Ala Pro Arg Tyr Thr Val His Thr Gly Ala Ala Arg Ser Cys Arg Phe
           355                 360                 365
   Arg Gly Arg His Tyr Lys Arg Glu Phe Arg Leu Glu Gly Glu Pro Val
       370                 375                 380
   Ala Leu Arg Cys Pro Gln Val Pro Tyr Trp Leu Trp Ala Ser Val Ser
   385                 390                 395                 400
   Pro Arg Ile Asn Leu Thr Trp His Lys Asn Asp Ser Ala Arg Thr Val
                   405                 410                 415
   Pro Gly Glu Glu Glu Thr Arg Met Trp Ala Gln Asp Gly Ala Leu Trp
               420                 425                 430
   Leu Leu Pro Ala Leu Gln Glu Asp Ser Gly Thr Tyr Val Cys Thr Thr
           435                 440                 445
   Arg Asn Ala Ser Tyr Cys Asp Lys Met Ser Ile Glu Leu Arg Val Phe
       450                 455                 460
   Glu Asn Thr Asp Ala Phe Leu Pro Phe Ile Ser Tyr Pro Gln Ile Leu
   465                 470                 475                 480
   Thr Leu Ser Thr Ser Gly Val Leu Val Cys Pro Asp Leu Ser Glu Phe
                   485                 490                 495
   Thr Arg Asp Lys Thr Asp Val Lys Ile Gln Trp Tyr Lys Asp Ser Leu
               500                 505                 510
   Leu Leu Asp Lys Asp Asn Glu Lys Phe Leu Ser Val Arg Gly Thr Thr
           515                 520                 525
   His Leu Leu Val His Asp Val Ala Leu Glu Asp Ala Gly Tyr Tyr Arg
           530                 535                 540
   Cys Val Leu Thr Phe Ala His Glu Gly Gln Gln Tyr Asn Ile Thr Arg
   545                 550                 555                 560
   Ser Ile Glu Leu Arg Ile Lys Lys Lys Lys Glu Glu Thr Ile Pro Val
                   565                 570                 575
   Ile Ile Ser Pro Leu Lys Thr Ile Ser Ala Ser Leu Gly Ser Arg Leu
               580                 585                 590
   Thr Ile Pro Cys Lys Val Phe Leu Gly Thr Gly Thr Pro Leu Thr Thr
           595                 600                 605
   Met Leu Trp Trp Thr Ala Asn Asp Thr His Ile Glu Ser Ala Tyr Pro
       610                 615                 620
   Gly Gly Arg Val Thr Glu Gly Pro Arg Gln Glu Tyr Ser Glu Asn Asn
   625                 630                 635                 640
   Glu Asn Tyr Ile Glu Val Pro Leu Ile Phe Asp Pro Val Thr Arg Glu
                   645                 650                 655
   Asp Leu His Met Asp Phe Lys Cys Val Val His Asn Thr Leu Ser Phe
               660                 665                 670
   Gln Thr Leu Arg Thr Thr Val Lys Glu Ala Ser Ser Thr Phe Ser Gly
           675                 680                 685
   Glu Ser Lys Tyr Gly Pro Pro Cys Pro Pro Cys Pro Ala Pro Glu Phe
       690                 695                 700
   Leu Gly Gly Pro Ser Val Phe Leu Phe Pro Pro Lys Pro Lys Asp Thr
   705                 710                 715                 720
   Leu Met Ile Ser Arg Thr Pro Glu Val Thr Cys Val Val Val Asp Val
                   725                 730                 735
   Ser Gln Glu Asp Pro Glu Val Gln Phe Asn Trp Tyr Val Asp Gly Val
               740                 745                 750
   Glu Val His Asn Ala Lys Thr Lys Pro Arg Glu Glu Gln Phe Asn Ser
           755                 760                 765
   Thr Tyr Arg Val Val Ser Val Leu Thr Val Leu His Gln Asp Trp Leu
       770                 775                 780
   Asn Gly Lys Glu Tyr Lys Cys Lys Val Ser Asn Lys Gly Leu Pro Ser
   785                 790                 795                 800
   Ser Ile Glu Lys Thr Ile Ser Lys Ala Lys Gly Gln Pro Arg Glu Pro
                   805                 810                 815
   Gln Val Tyr Thr Leu Pro Pro Ser Gln Glu Glu Met Thr Lys Asn Gln
               820                 825                 830
   Val Ser Leu Thr Cys Leu Val Lys Gly Phe Tyr Pro Ser Asp Ile Ala
           835                 840                 845
   Val Glu Trp Glu Ser Asn Gly Gln Pro Glu Asn Asn Tyr Lys Thr Thr
       850                 855                 860
   Pro Pro Val Leu Asp Ser Asp Gly Ser Phe Phe Leu Tyr Ser Arg Leu
   865                 870                 875                 880
   Thr Val Asp Lys Ser Arg Trp Gln Glu Gly Asn Val Phe Ser Cys Ser
                   885                 890                 895
   Val Met His Glu Ala Leu His Asn His Tyr Thr Gln Lys Ser Leu Ser
               900                 905                 910
   Leu Ser Leu Gly Lys
           915